(19) Europäisches Patentamt / European Patent Office / Office européen des brevets

(11) **EP 3 502 266 A1**

(12) **EUROPEAN PATENT APPLICATION**

(43) Date of publication:
**26.06.2019 Bulletin 2019/26**

(21) Application number: **17210008.3**

(22) Date of filing: **22.12.2017**

(51) Int Cl.:
*C12P 21/02* (2006.01)      *C12R 1/125* (2006.01)
*C07K 7/06* (2006.01)        *C07K 7/64* (2006.01)
*C07K 9/00* (2006.01)        *A01N 63/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicants:
- **VITO NV**
  **2400 Mol (BE)**
- **Quaid-i-Azam University**
  **45320 Islamabad (PK)**

(72) Inventors:
- **Bastiaens, Leen**
  **2400 Mol (BE)**
- **Simons, Queenie**
  **2400 Mol (BE)**
- **Van Roy, Sandra**
  **2400 Mol (BE)**
- **Ahmed, Safia**
  **45320 Islamabad (PK)**
- **Qazi, Muneer Ahmad**
  **45320 Islamabad (PK)**
- **Naeem, Afshan Hina**
  **45320 Islamabad (PK)**
- **Umer, Aiman**
  **45320 Islamabad (PK)**

(74) Representative: **V.O.**
**P.O. Box 87930**
**Carnegieplein 5**
**2508 DH Den Haag (NL)**

(54) **BIOSURFACTANT PRODUCTION**

(57) The present invention relates to a newly identified bio-surfactant producing bacterial strain, Bacillus subtilis QVS1, which was deposited under accession number LMG P-30406, and to fermentative processes wherein such a strain is employed for surfactin and fengycin (cyclic lipopeptides)-containing biosurfactant production, to crude biosurfactant mixtures or preparations obtained by such processes, and to advantageous culture media comprising red beans and culture conditions for bio-surfactant producing micro-organisms.

**Description**

FIELD OF THE INVENTION

The present invention is in the field of biochemistry and biotechnology. More specifically, the invention is in the field of biosurfactant production by employing biosurfactant-producing micro-organisms such as *Bacillus subtilis* in fermentative processes. The invention relates to a newly identified biosurfactant producing bacterial strain, to fermentative processes wherein such a strain is employed for biosurfactant production, to crude biosurfactant mixtures or preparations obtained by the above-mentioned processes, and to advantageous culture media and culture conditions for biosurfactant-producing microorganisms.

STATE OF THE ART

The worldwide production of surfactants is estimated in 18 million tonnes per year, with 3 million tonnes being produced in Western Europe. Surfactants are mainly manufactured from petrochemical feed stocks, and only 25% are produced from renewable resources. Their synthesis requires hazardous chemicals and is associated to environmental problems due to inherent toxicity. The biodegradability of commercially available surfactants has been improved, but not fully achieved. The need to use renewable resources to produce surfactants and replace petrochemical products has led to the increasing use of oleo-chemicals, which are mostly obtained from conventional agricultural practices and are increasingly being associated with a negative environmental impact.

Biosurfactants are amphiphilic compounds with emulsifying, wetting, solubilizing, detergent and phase-dispersing properties that are produced by living organisms, predominantly microorganisms. Biosurfactants have gained considerable interest in recent years due to their low toxicity, biodegradable nature and diversity, which makes them superior to chemical surfactants. A further advantage is that they can be produced from renewable resources. These unique properties allow their use in industrial operations and have the potential to replace chemically-derived surfactants from oleo-chemical feed stocks. Types of biosurfactants include glycolipids, lipopeptides, lipoproteins, fatty acids, neutral lipids and phospholipids (Lang S., Curr Opin Colloid Interface Sci, 7:12-20 (2002)).

Lipopeptides are amongst the most efficient surfactants (Zajic JE et al., CRC Crit Rev Biotechnol, 1:87-107 (1984)). They are composed of peptides linked to fatty acids, with the peptide moiety often being cyclic and either being neutral or having a negative charge. The best characterized lipopeptides are those produced by species of *Bacillus*, which may include surfactin, iturin, fengycin, lichenysin, mycosubtilin and bacilomycin (Maier RM., Adv Appl Microbiol, 52:101-21 (2003); Steller S et al., J Chromatogr B, 737:267-75 (2000)). Surfactin, produced by various strains of *Bacillus subtilis* (Lin et al., Biotechnol. Prog., 9:138-145 (1993)), is recognized as one of the most effective biosurfactants (Grangemard et al., Appl. Biochem. Biotechnol., 90, 199-210 (2001). The primary structure of surfactin consists of a cyclic lipopeptide consisting of seven amino acids bonded to a hydrophobic fatty acid chain of 13 to 16 carbons.

In order to replace chemical surfactants by biosurfactants, one strategy is to identify new biosurfactant-producing micro-organisms. A distinction needs to be made between new wild-type environmental isolates and new strains that are a result from genetic recombination (GMO). Although genetic modified strains may in some instances have a higher yield, wild-type strains to generate non-GMO-based products are preferred by end-users and customers. It is difficult to identify novel wild-type biosurfactant-producing micro-organisms, especially micro-organisms that are appropriate for use in large-scale fermentation processes for biosurfactant production. Such isolates need to satisfy multiple requirements. These requirements include production of appropriate amounts of biosurfactant under culture conditions that are as economically favorable as possible, and should further produce a mixture of biosurfactants with favorable emulsification and surface tension properties, and preferably be stable and therefore functional in environmental conditions where temperature, pH or salinity strongly fluctuate.

In the same manner, it is equally important to devise culture conditions that allow for improved production of biosurfactants, both in terms of quantity and quality, and which conditions are economically favorable such that they allow for scale-up. For instance, currently, yeast extract (also referred to as "YE") is employed as a growth substrate for biosurfactant-producing micro-organisms. The disadvantage of using yeast extract in culture media is that it is relatively expensive. Cheaper growth substrates would make the biosurfactant production process economically more favorable.

A first aim of the invention is to provide a new wild-type biosurfactant-producing micro-organism, which is capable of producing appropriate amounts of biosurfactant under economically favorable culture conditions. In addition, such a micro-organism is able to produce a mixture of biosurfactants that is stable and therefore functional under environmental conditions where temperature, pH and salinity fluctuate. A second aim of this invention is to identify growth substrates for biosurfactant-producing micro-organisms, alternative to yeast extract, which are cheaper than yeast extract (economically more favorable) while not giving in on biosurfactant production yield. In addition, it is a further aim that such alternative growth substrates are compatible with the newly identified wild-type biosurfactant-producing micro-organism.

THE INVENTION

Unexpectedly, a new biosurfactant-producing *Bacillus subtilis* strain was discovered near an oilfield in Pakistan. This strain, also referred to as *"Bacillus subtilis* QVS1" or "QVS1", has been deposited with the Belgian Coordinated Collections of Microorganisms (BCCM)/LMG (hereinafter: BCCM/LMG), Ghent, Belgium, under Accession Number "LMG P-30406" on 30 November 2017.

Therefore, the present invention provides a *Bacillus subtilis* (strain) deposited with the BCCM/LMG, Ghent, Belgium, under Acc. No. LMG P-30406, or a mutant *Bacillus subtilis* thereof.

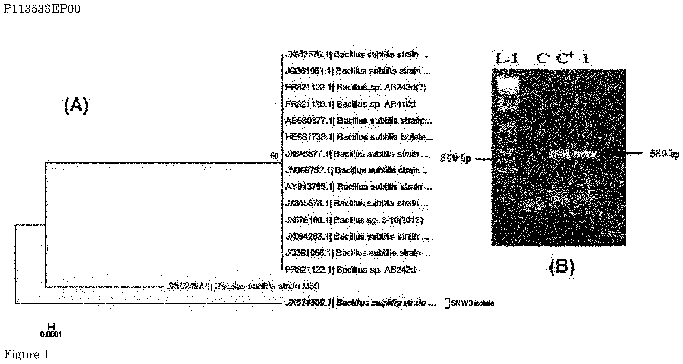

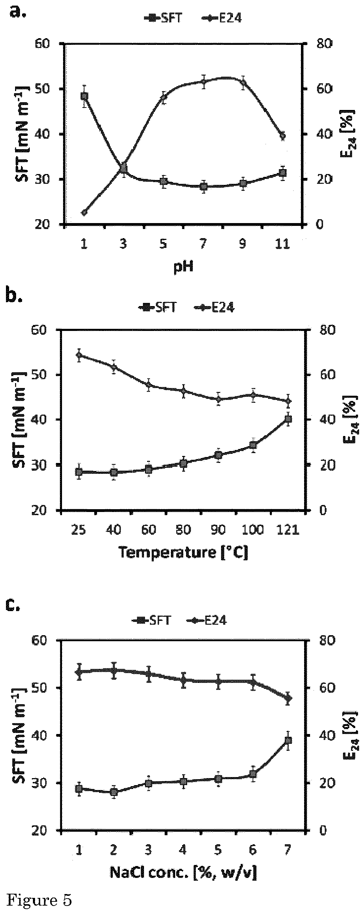

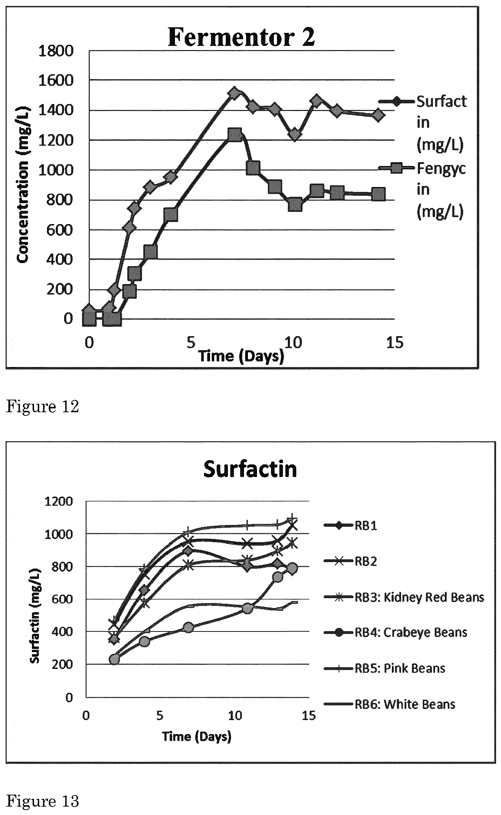

The deposited bacterium was isolated and characterized as described in Example 1 and *inter alia* shown in Figures 1A and 1B, which together led to the conclusion that this bacterium belongs to the species of *Bacillus subtilis.* A *Bacillus subtilis* of the invention was found to produce appropriate amounts of biosurfactants, preferably lipopeptides, resulting under certain culture conditions in combined surfactin and fengycin yields of at least 2.6 g/L (Figure 12). Surprisingly, a *Bacillus subtilis* of the invention was found to produce a mixture of biosurfactants that is highly stable under fluctuating environmental conditions, as shown in Figure 5.

A *Bacillus subtilis* of the invention is, in the context of its deposit, in the form of an isolated pure culture, i.e. a (pure) bacterial isolate. The term "bacterial isolate", as used herein, refers to a strain of bacteria which is separated from a mixed bacterial culture and is substantially or essentially free from components such as other microorganisms that normally accompany it in its native state, e.g., in a culture.

It is noted that a *Bacillus subtilis* of the invention may be comprised in an inoculum or in a culture medium, i.e. an inoculated culture medium, the latter optionally also comprising other biosurfactant-producing micro-organisms.

The term *"Bacillus subtilis"*, as used herein, refers to a bacterium belonging to the species of *Bacillus subtilis.* Bacteria of the *Bacillus subtilis* species are generally Gram-positive, catalase-positive and rod-shaped bacteria, and have the propensity to form a tough, protective endospore under certain environmental conditions. The term *"Bacillus subtilis* of the invention", as herein, also includes reference to progeny of such a *Bacillus subtilis.*

The term "QVS1", as used herein, may in some instances also be referred to as "SNW3".

A *Bacillus subtilis* of the invention is preferably identifiable by a 16S rRNA gene sequence comprising the nucleic acid sequence of SEQ ID NO:1. Thus, a *Bacillus subtilis* of the present invention preferably has a 16S rRNA gene sequence comprising the nucleic acid sequence of SEQ ID NO:1. Preferably, a *Bacillus subtilis* of the invention is halo-tolerant and thermo-tolerant.

The invention also provides mutants of the deposited *Bacillus subtilis* of the invention. The term "mutant", as used herein, refers to a bacterium or strain derived, or a bacterium or strain which can be derived, from the deposited *Bacillus subtilis* of the invention by means of e.g. genetic engineering, radiation and/or chemical treatment. The mutant can also be a spontaneously occurring mutant.

Preferably, the mutant is a functionally equivalent mutant, e.g. a mutant that is, except for one or more alterations in its genomic DNA sequence - preferably conservative mutations or mutations not changing the encoded amino acid sequences - not distinguishable from the deposited *Bacillus subtilis* of the invention in terms of morphological, biochemical and/or functional characteristics such as a biosurfactant production pattern at a predetermined moment in time, a biosurfactant biosynthetic pathway and/or stability pattern of the biosurfactants produced. These patterns, and other functional characteristics of the deposited *Bacillus subtilis,* are extensively described herein for the deposited bacterium, and preferably also apply to a *Bacillus subtilis* mutant of the invention. The skilled person is well aware of method and means to compare biosurfactant production patterns of the two strains. One way would be to culture such strains under the same culture conditions, and subsequently analyze the cell-free culture medium (after culturing) or surfactant, which may be partially purified, by for instance Thin Layer Chromatography (TLC), Fourier Transform Infra-Red-Attenuated Total Reflectance (FTIR-ATR), liquid chromatography and/or mass spectrometry as for instance described herein, and compare the biosurfactant patterns obtained. Also described herein are test for establishing stability of the biosurfactants produced, which provide the skilled person with the methods and means to compare two bacterial strains for stability of the biosurfactants produced.

The invention also provides a use of a *Bacillus subtilis* of the invention for producing biosurfactants.

Further, the invention also provides a method for producing biosurfactants, comprising the step of: a) culturing a *Bacillus subtilis* of the invention in a culture medium under culture conditions that allow for the production of biosurfactants; b) optionally, after step a), separating from said culture medium a *Bacillus subtilis* of the invention so as to provide a cell-free culture medium comprising biosurfactants; and c) optionally, recovering biosurfactants from said cell-free culture medium.

The term "biosurfactants", as used herein, refers to amphiphilic compounds with emulsifying, wetting, solubilizing, detergent and/or phase-dispersing properties that are produced by living organisms, predominantly micro-organisms such as bacteria. Types of biosurfactants include glycolipids, lipopeptides, lipoproteins, fatty acids, neutral lipids and phospholipids.

Preferably, the biosurfactant is a lipopeptide. Lipopeptides are composed of peptides linked to fatty acids, with the peptide moiety often being cyclic and either being neutral or having a negative charge. Preferably, the lipopeptide is selected from the group formed by surfactin, iturin, fengycin, lichenysin, mycosubtilin and bacilomycin. More preferably, the lipopeptide is surfactin, such as surfactin-C13, surfactin-C14 and/or surfactin-C15, and fengycin, such as fengycin A and/or B.

It was established that a *Bacillus subtilis* of the invention is capable of producing at least surfactin and fengycin (*inter alia* Figure 12). Under certain culture conditions, combined yields of at least 2.6 g/L were achieved, with surfactin yields peaking around 1.5 g/L (Figure 12).

The term "culturing", as used herein, refers to the propagation of a micro-organism on or in media of various kinds. In the context of the invention, the term preferably includes reference to a step of fermenting a growth substrate in a culture medium into a biosurfactant.

The term "fermentation" or "fermenting", as used herein, refers to a process in which one or more (growth) substrates present in a culture medium or fermentation medium, which terms can be used interchangeably herein, are converted by a microorganism into a product, i.e. a biosurfactant. In the context of the invention, fermentation preferably occurs in the presence of oxygen, and is thus an aerobic fermentative method.

The term "fermentation medium" or "culture medium", as used herein, refers to the environment in which fermentation is carried out and which includes the fermentation of growth substrate(s), such as the carbohydrate source that is metabolized by the biosurfactant-producing microorganism. Furthermore, the "fermentation medium" may comprise nutrients and/or growth stimulators for the fermenting microorganism(s). Nutrient and growth stimulators are widely used in the art of fermentation and include nitrogen sources, such as ammonia, urea, vitamins and minerals, or combinations thereof.

Preferably, the culture medium is a liquid culture medium. The terms "culture medium", "fermentation medium" and "growth medium" are used interchangeably herein. The skilled person is well aware of appropriate culture media for micro-organisms, especially for well-known bacteria such as *Bacillus subtilis.* An example of a base growth medium for *Bacillus subtilis* is mineral salts medium (MSM), as for instance defined in Examples 1 and 2 herein. Such a medium may be supplemented with carbon (carbohydrate), nitrogen and/or phosphorus sources, and may be further supplemented with growth inducers.

The phrase, "culture conditions that allow for the production of biosurfactants", as used herein, refers to culture conditions under which a measurable amount of biosurfactant is produced. Relevant culture conditions are temperature, pH and agitation speed for cultures in liquid media. In addition, the composition of the culture medium may play a role on biosurfactant production. As is evident from *inter alia* Figures 1 and 8-13, almost all culture conditions allow for the production of a measurable amount of biosurfactant. It is thus within the capabilities of the skilled person to set culture conditions under which at least some amount of biosurfactant, even be it a low but measurable amount, is produced.

As a culture condition, the temperature can be 27-40°C, preferably 28-37°C, more preferably 29-37°C, most preferably 29-33°C or about 30°C. As a culture condition, the pH is preferably 5-10 or 6-9, more preferably 7-8, most preferably about 7 or about 8. As a culture condition, the agitation speed is preferably 100-200 rounds per minute (rpm), more preferably 125-175 rpm, even more preferably 140-160 rpm, most preferably about 150 rpm. These culture conditions allow for beneficial biosurfactant production by a *Bacillus subtilis* of the present invention (Fig. 1A-1C). Alternatively, or in addition, it was established that yeast extract, especially at a concentration of at least 1.75% or at least 2% (w/v), allowed for further improvement in biosurfactant production by a *Bacillus subtilis* of the present invention. Alternatively, or in addition, it was established that especially urea, preferably in concentration of at least 0.1% (w/v) employed as a nitrogen source in the culture medium, provides for further improvement in biosurfactant production by a *Bacillus subtilis* of the present invention.

Unless indicated otherwise, wt.% or % (w/v), as referred to herein, are calculated over the total weight or volume of the culture medium before or after, preferably before, inoculation of the culture medium with a *Bacillus subtilis* of the present invention.

Preferably, after culturing a *Bacillus subtilis* of the invention according to step a) of a method of the invention, the *Bacillus subtilis* of the invention is separated from the culture medium so as to provide a cell-free culture medium comprising biosurfactants. The cell-free culture medium comprising biosurfactants may also be referred to as supernatant. If further biosurfactant-producing micro-organisms are employed in the culturing step, such micro-organisms are preferably also separated from the culture medium.

The term "supernatant" or "cell-free culture medium", as used interchangeably herein, refers to the liquid broth remaining when cells grown in the culture medium are removed by centrifugation, filtration, sedimentation, or other means well known in the art. In the context of the invention, such a liquid broth comprises biosurfactants and is of commercial interest. Centrifugation methods for separating supernatant from bacterial cells include, but are not limited to, for instance a step of centrifugating the culture broth obtained after aforementioned step a) at 12000 rpm (for 15 min, at 4 °C) followed by filtration (for instance over a 0.45 μm syringe filter).

In other words, the step of separating from said culture medium a *Bacillus subtilis* of the invention, may also be phrased as b) providing, after step a), a supernatant comprising biosurfactants. The supernatant may also be referred to herein as cell-free supernatant (CFS).

As a further step, a method for producing biosurfactants of the invention preferably includes c) recovering biosurfactants from the cell-free culture medium. Recovery strategies for biosurfactants are generally known in the art, and include for instance acid preparation, foam fractionation or combinations thereof (Desai et al., Microbiology and Molecular Biology Reviews, 61:47-64 (1997)). Alternatively, (organic) solvent extraction can be applied by a skilled person to recover, or purify, biosurfactants from a cell-free culture medium comprising biosurfactants. A specific example of organic solvent extraction involves a combination of chloroform and methanol, which may be formulated in different ratios so as to regulate the polarity adjustment of the extractant to a desired level. Chloroform, however, has as a disadvantage in that it is toxic.

Preferably, in a method of the invention, recovery, or (partial) purification, is performed by acid precipitation, followed by solvent extraction. Acid precipitation can be performed by acidifying cell-free culture medium to a pH of 1-4, preferably about 2, with for instance HCl and leaving it for a couple of hours, for instance overnight, at a temperature between 2-10 °C, preferably about 4 °C. The precipitate can be collected by centrifuging the acidified biosurfactant-containing culture medium, and preferably re-dissolving the precipitate in an appropriate buffer.

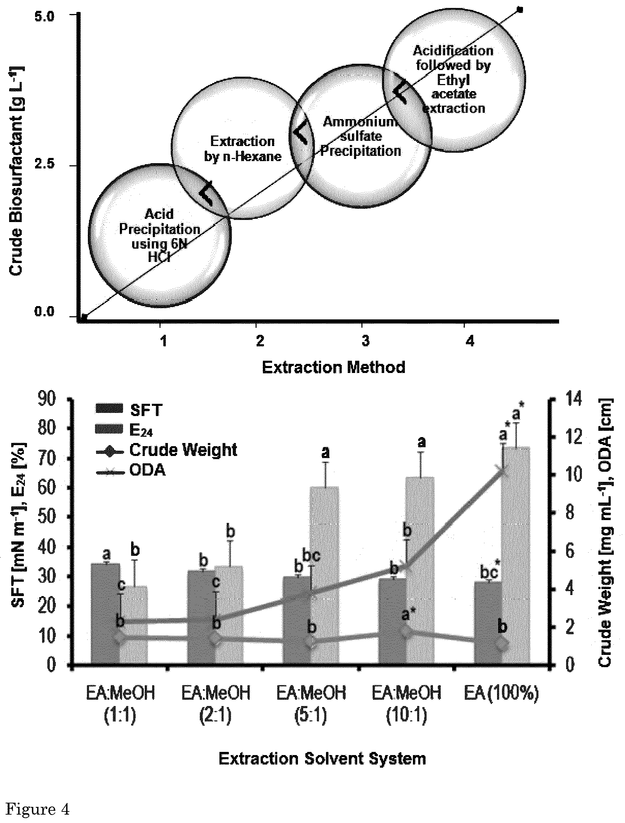

Preferably, the collected precipitate is subsequently subjected to solvent extraction with ethyl acetate. It was found that ethyl acetate can be beneficially employed in biosurfactant recovery, since both maximum crude weight and maximum biosurfactant concentration indicated by oil displacement assay (ODA) was achieved when ethyl acetate was employed as an extraction solvent. More preferably, the solvent extraction is performed with either (i) ethyl acetate in combination with methanol, preferably in a ratio of about 2:1, (v/v), respectively, so as to optimize for extracted crude weight, or (ii) ethyl acetate as sole extraction solvent so as to optimize for specificity towards biosurfactant extraction, which is for instance indicated by an increased oil displacement assay (ODA) value *(vide* Figure 4).

Recovery, or harvesting of biosurfactants, preferably occurs 1-20 days, more preferably 5-15 days, most preferably 7-10 days or 8-9 days, after inoculation of a *Bacillus subtilis* of the invention to a culture medium as described herein. Such a harvesting time is beneficially employed in a batchwise process. Continuous harvesting is also envisaged as an alternative to a batchwise process for biosurfactant production. Preferably, a method of the invention provide for the production of surfactin and/or fengycin in a concentration of at least 2 g/l, more preferably at least 2.5 g/l, as measurable in the cell-free culture medium comprising biosurfactants.

Further, a method of the invention is preferably a method for producing biosurfactants on an industrial scale.

In general, a method for producing biosurfactants of the invention can be performed in a reactor or fermentor for producing biosurfactants. Such reactors or fermenters are generally known in the art, and include for instance shake flasks or other containers that comprise a culture volume for culturing biosurfactant-producing micro-organisms. A non-limiting example of such a reactor is the BIOSTAT® D fermentor system (Sartorius BBI Systems GmbH, DE). The present invention therefore also provides a (bio)reactor or fermentor for the production of biosurfactants, comprising (i) a *Bacillus subtilis* of the invention, (ii) a culture medium of the invention, (iii) a culture medium of the invention comprising a biosurfactant-producing micro-organism, or (iv) a culture medium for the production of biosurfactants by *Bacillus subtilis* bacteria, comprising a *Bacillus subtilis* of the invention.

In the same manner, the invention also provides a culture medium for culturing or growing a biosurfactant-producing micro-organism, comprising a *Bacillus subtilis* of the invention.

The invention also relates to economically favorable culture media, or growth substrates, for biosurfactant-producing micro-organisms. Such growth substrates are cheaper than for instance the commonly applied yeast extract substrate, without giving in on biosurfactant production yield. A culture medium of the invention may even provide for improved biosurfactant production as compared to for instance a yeast extract-based culture medium.

To that extent, the invention provides a culture medium comprising beans, preferably red beans, said medium optionally further comprising a starch. Said culture medium is a culture medium for culturing or growing biosurfactant-producing micro-organisms.

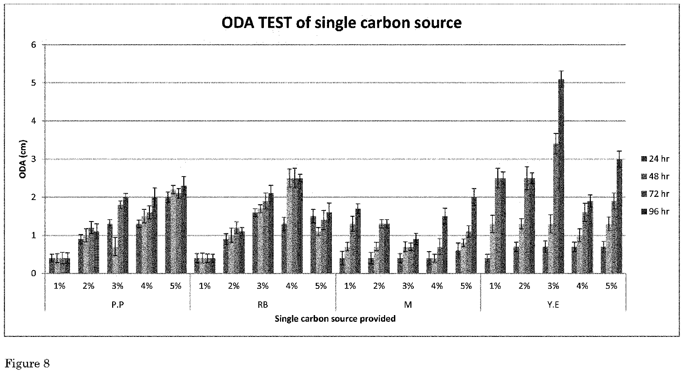

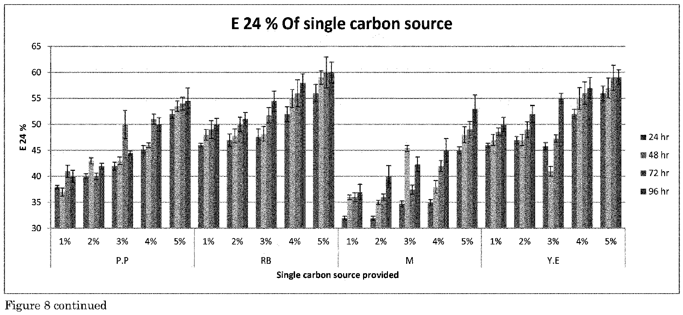

It was found that a beans based culture medium is highly advantageous when growing biosurfactant-producing micro-organisms, which is evident from *inter alia* Figures 8-11 and Example 2.

Preferably, a culture medium of the invention is a liquid culture medium. The liquid culture medium is preferably a water-based culture medium. A suitable aqueous base liquid for such a medium is ionized or deionized water. Aqueous base liquids for the growth of biosurfactant-producing micro-organisms are generally known in the art. A preferred example of such an aqueous base liquid in a culture medium as described herein is a mineral salts medium, which comprises deionized water, and 1, 2, 3, 4, 5 or 6 salts selected from the group formed by $Na_2HPO_4$, $KH_2PO_4$, $MgSO_4 \cdot 7H_2O$, $FeSO_4 \cdot 7H_2O$, NaCl and $CaCl_2$. Such a mineral salts medium preferably further comprise 1, 2, 3, 4, 5 or 6 trace elements selected from the group formed by $ZnSO_4 \cdot 7H_2O$, $MnSO_4 \cdot 4H_2O$, $H_3BO_3$, $CuSO_4 \cdot 5H_2O$, $NH_4MoO_4 \cdot 2H_2O$ and KI. The pH of such a culture medium may vary, but is preferably 6-9, more preferably 7-8, most preferably about 7 or about 8. Another example of a culture medium for cultivation of *Bacillus subtilis* is ATCC® Medium 3: Nutrient agar or nutrient broth.

The term "bean" or "beans", as used herein, refers to a seed of one of several genera of the plant family *Fabaceae*, which are *inter alia* used for human or animal food. Preferably, the bean is produced by, or originates from, a plant of the genus *Phaseolus*, *Vigna* or *Abrus*. More preferably, the bean is produced by, or originates from, a plant of the species *Phaseolus vulgaris*, *Vigna angularis*, *Vigna umbellata* or *Abrus precatorius*. Most preferably, the bean is a red bean and is produced by, or originates from, a plant of the aforementioned species. In instances where the term "bean(s)" is used herein, preferably reference is made to red bean(s). The term "bean" or "beans", as used herein, also includes reference to processed beans such as a flour or powder of beans.

The term "red bean(s)", also referred to as "RB", as used herein, refers to light-red (including pink) to dark-red or dark colored beans produced by, or originating from, a plant of the family *Fabaceae*. More preferably, such beans are produced by, or originate from, a plant of the genus *Phaseolus*, *Vigna* or *Abrus*. More preferably, such a bean is produced by, or originates from, a plant of the species *Phaseolus vulgaris*, *Vigna angularis*, *Vigna umbellata* or *Abrus precatorius*. Such beans can be speckled or non-speckled. Examples of red beans that find application with the present invention are red beans belonging to *Phaseolus vulgaris*, *Phaseola Vigna* (i.e. *Vigna angularis* (adzuki bean), *Vigna umbellata* (rice bean) or *Fabacea Abrus* (i.e. *Abrus precatorius* (crab eye bean); in particular selected from the group comprising adzuki bean, ricebean, crab eye bean, pink bean and cranberry red bean.

Preferably, the beans are processed into a flour or powder before incorporation in the culture medium. In other words, a culture medium of the invention preferably comprises a flour or powder of beans. Such a flour may comprise bean particles in mm size, or even smaller such as $\mu$m-mm size. The skilled person is aware of method and means to provide flours from products such as beans, such as for instance by blender-mixing, allowing for a size reduction to mm size, and by subsequent freezing, freeze-drying and grinding to powder size.

Preferably, a culture medium of the invention comprises at least 0.01, 0.02, 0.05, 0.1, 0.2, 0.5, 1, 2, 3, 4, 5, 6, 7, 8 or 10 wt.% beans. More preferably, a culture medium of the invention comprises at least 2 wt.% or a least 3 wt.% of beans. Alternatively, a culture medium of the invention comprises 0.1-40 wt.%, preferably 0.2-15 wt.%, more preferably 1-10 wt.%, most preferably 2-6 or about 3% or about 6%, of beans.

The term "starch", as used herein, refers to any starch of natural origin whether processed, chemically modified or treated, including starches such as for example wheat starch, corn starch, potato starch, and rice starch. Starch can also be derived from plant sources such as cassava, tapioca, and pea. It is a polysaccharide that comprises a blend of amylose and amylopectin. Preferably, the starch is a potato starch (also referred to as "PS"), such as available from Sigma-Aldrich (S4251).

The starch, when incorporated in the culture medium, is preferably in the form of a powder or flour.

Preferably, a culture medium of the invention comprises at least 0.001, 0.01, 0.02, 0.05, 0.1, 0.2, 0.3, 0.4, 0.5, 0.8, 1, 2, 3 wt.% of starch. More preferably, a culture medium of the invention comprises at least 0.2, most preferably at least, or about, 0.5 wt.% of starch. Alternatively, a culture medium of the invention comprises 0.01-25 wt.%, preferably 0.1-10 wt.%, more preferably 0.2-5 wt.%, most preferably 0.3-1 wt.% of starch.

A culture medium of the invention is preferably further supplemented with a growth substrate serving as a nitrogen source.

The term "nitrogen source", as used herein, refers to a compound (organic or inorganic), and preferably inorganic or non-protein, that contains one or more nitrogen atoms and is for use as a growth substrate in growth media for culturing micro-organisms. Examples such nitrogen sources that find application with the invention are ammonia, nitrate and/or urea, preferably urea (also referred to herein as "U").

Preferably, a culture medium of the invention comprises at least 0.001, 0.003, 0.005, 0.007, 0.1, 0.2, 0.3, 0.4, 0.5, 0.7 or 1 wt.% of urea. More preferably, a culture medium of the invention comprises about 0.4 wt.% of urea.

A culture medium of the invention is preferably further supplemented with a growth substrate serving as a phosphorus source.

The term" phosphorus source", as used herein, refers to an organic or inorganic compound, preferably inorganic, that contains one or more phosphorus atoms and is for use as a growth substrate in growth media for culturing micro-organisms. The skilled person is aware of such phosphorus sources, which may suitable include phosphate included as a phosphate buffer in an aqueous base liquid as referred to herein.

Alternatively, or in addition, a culture medium of the invention can be defined by reference to a C:N ratio of the components in the culture medium. A person skilled in the art is aware of how such a C:N ratio is measured, for instance via elemental analysis through the well-established Dumas-method. Preferably, such a culture medium of the invention has a C:N ratio of 1-100, preferable 2-60, more preferably 3-10 or 6.5-7.5. A culture medium of the invention most preferably has a C:N:P ratio of about 10:3:1, respectively.

The most preferred culture medium of the invention comprises about 60 g/l beans, preferably red beans, about 5 g/l potato starch and about 4 g/l urea. In other words, a culture medium of the invention most preferably comprises about 6 wt.% beans, about 0.5 wt.% potato starch and about 0.4 wt.% urea.

Further, a culture medium of the invention may comprises further growth supplements or growth inducers, such as yeast extract and amino acids such as alanine, preferably in a concentration of 0.1-0.5% (w/v). Other known growth inducers are for instance spores of metals and vitamins.

Energy sources, such as carbohydrate (carbon) sources, nitrogen sources and phosphorus sources, can be separately added to the culture medium, or they can be incorporated in the culture medium simultaneously. Such energy sources are preferably sterilized before being incorporated in the culture medium.

A culture medium of the invention as described in this section is preferably employed in a method for producing biosurfactants of the invention. Reference to a culture medium throughout this text may include reference to a culture medium of the invention for the purpose of combinability of embodiments.

The invention also provides a culture medium of the invention comprising a biosurfactant-producing micro-organism.

The term "biosurfactant-producing micro-organism", as used herein, refers to an unicellular eukaryotic organism such as yeasts, microalgae and fungi, or a prokaryotic organism such as bacteria, which are capable of producing biosurfactants. A preferred biosurfactant-producing micro-organism is a bacterium, more preferably a *Bacillus subtilis,* most preferably a *Bacillus subtilis* of the invention.

In another aspect, the invention provides a method for producing biosurfactants, comprising the step of: a) culturing a biosurfactant-producing micro-organism in a culture medium of the invention, under culture conditions that allow for the production of biosurfactants; b) optionally, after step a), separating from said culture medium the biosurfactant-producing micro-organism so as to provide a cell-free culture medium comprising biosurfactants; c) optionally, recovering biosurfactants from said cell-free culture medium.

The invention also provides a use of beans and starch in a culture medium of a biosurfactant-producing micro-organism for producing biosurfactants. Alternatively, the invention provides a use of a culture medium comprising beans and starch for producing biosurfactant.

The invention also provides a composition comprising biosurfactants, obtainable by a method for producing biosurfactants of the invention. Such a composition may also be referred to as a biosurfactant composition obtainable by a method for producing biosurfactants of the invention.

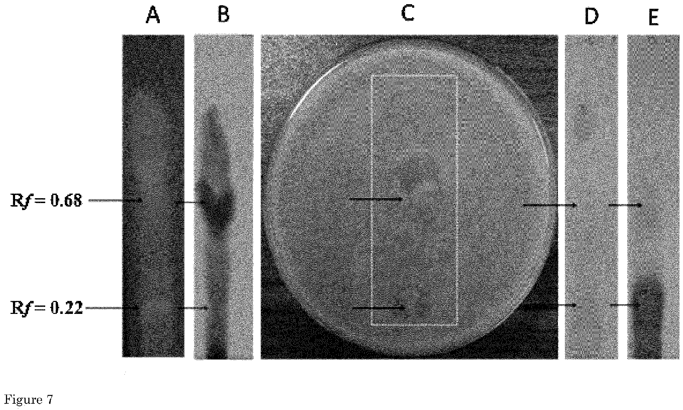

Unexpectedly, it was found that biosurfactants produced by the deposited *Bacillus subtilis* strain are highly stable, in terms of emulsifying activity and surface tension reduction, under environmental conditions where temperature, pH and salinity strongly fluctuate (Figure 5). This beneficially allows for employing biosurfactants of the invention in a broad range of applications, such as bioremediation of oil contaminated marine environments, where marine salinity can be as high as 3%. In addition, it was found that a biosurfactant composition of the invention exhibits antibacterial activity against methicillin-resistant *Staphylococcus aureus* (MRSA) (Figure 7).

A composition of the invention preferably comprises at least surfactin and fengycin.

Such a composition can be (i) a culture broth obtained after performing step a) of a method for producing biosurfactants of the invention, (ii) a cell-free culture medium, or supernatant, obtained after performing steps a)-b) of a method for producing biosurfactants of the invention, or (iii) the recovered, or extracted, composition obtained after performing steps a)-c) of a method for producing biosurfactants of the invention.

Preferably, a composition of the invention is at least partially purified, in that at least one of steps b)-c) of a method of the invention are performed. More preferably, a composition of the invention is a crude, or crude extract, which can be obtained with recovery procedures as described hereinabove.

Preferably, a composition of the invention has, or provides for, an:

(i) emulsification index ($E_{24}$) of at least 40%, preferably at least 50%, over a pH range of 4-9 or 5-9, and a surface tension of at most 40 mN m$^{-1}$, preferably at most 35 mN m$^{-1}$, more preferably at most 32 mN m$^{-1}$ over a pH range of 3-11 or 5-9;

(ii) emulsification index ($E_{24}$) of at least 40%, preferably at least 45%, more preferably at least 50%, over a temperature range of 25-121 °C or 25-100 °C, and a surface tension of at most 35 mN m$^{-1}$ over a temperature range of 25-100 °C; and

(iii) emulsification index ($E_{24}$) of at least 50% over a NaCl concentration range of 1-7% (w/v), and a surface tension of at most 33 mN m$^{-1}$ over a NaCl concentration range of 1-6% (w/v).

"Over a range" means that the recited emulsification index and surface tension values are achieved for every value in the recited range.

Emulsification index and surface tension measurement techniques are generally known in the art. Preferably, in the context of the invention, emulsification index and surface tension are measured as described in Example 1 or Example 2.

Preferably, a composition of the invention provides for an emulsification index and surface tension value of 67-69% and 28-29 mN m$^{-1}$, respectively, when the pH, temperature and salinity are 7.0, 25 °C and 2% (w/v), respectively.

Further, in terms of biosurfactant production pattern or characteristics, it was found by applying ultra high performance liquid chromatography (UHPLC) in combination with static secondary ion mass spectrometry (SSIMS), that the deposited strain has the propensity to produce a composition comprising surfactin-C13, surfactin-C14 and surfactin-C15, and fengycin A and B, preferably as dominating or peak (in terms of quantity) biosurfactants or lipopetides in said composition (data not shown).

Further, a composition of the invention preferably has a critical micelle concentration (CMC) of at least 600 mg/ml or about 610 mg/ml. CMC measurement techniques are generally known in the art. Preferably, in the context of the invention, CMC is measured as described in Example 1. Further, multiple applications of a composition of the invention are foreseen, especially given its ability to be functional under varying environmental conditions.

Therefore, the invention provides a use of a composition of the invention in bioremediation of oil-contaminated environments, such as marine environments.

The invention further provides a bactericidal or bacteriostatic medicament, comprising a composition of the invention. The invention also provides a composition of the invention for use as a medicament. Preferably, said composition is for use in the treatment of a bacterial infection in a subject, preferably an MRSA infection.

For the purpose of clarity and a concise description, features are described herein as part of the same or separate aspects and preferred embodiments thereof, however, it will be appreciated that the scope of the invention may include embodiments having combinations of all or some of the features described.

The invention will now be illustrated by the following examples, which are provided by way of illustration and not of limitation and it will be understood that many variations in the methods described and the amounts indicated can be made without departing from the spirit of the invention and the scope of the appended claims.

The content of the documents referred to herein is incorporated by reference.

**SEQ ID NO:1.** *Bacillus subtilis* strain SNW3 16S ribosomal RNA gene, partial sequence (Genbank Acc. No.: JX534509.1):

```
GTGGGCCGTAAGGGCTCGCAGGCGGTTTCTTAAGTCTGATGTGAAAGCCCCCGGCTCAA

CCGGGGAGGGTCATTGGAAACTGGGGAACTTGAGTGCAGAAGAGGAGAATGGAATTCCA

CGTGTAGCGGTGAAATGCGTAGAGATGTGGAGGAACACCAGTGGCGAAGGCGACTCTCT

GGTCTGTAACTGACGCTGAGGAGCGAAAGCGTGGGGAGCGAACAGGATTAGATACCCTG

GTAGTCCACGCCGTAAACGATGAGTGCTAAGTGTTAGGGGGTTTCCGCCCCTTAGTGCT

GCAGCTAACGCATTAAGCACTCCGCCTGGGGAGTACGGTCGCAAGACTGAAACTCAAAG

GAATTGACGGGGGCCCGCACAAGCGGTGGAGCATGTGGTTTAATTCGAAGCAACGCGAA

GAACCTTACCAGGTCTTGACATCCTCTGACAATCCTAGAGATAGGACGTCCCCTTCGGG

GGCAGAGTGACAGGTGGTGCATGGTTGTCGTCAGCTCGTGTCGTGAGATGTTGGGTTAA

GTCCCGCAACGAGCGCAACCCTTGATCTTAGTTGCCAGCATTCAGTTGGGCACTCTAAG

GTGACTGCCGGTGACAAACCGGAGGAAGGTGGGGATGACGTCAAATCATCATGCCCCTT

ATGACCTGGGCTACACACGTGCTACAATGGACAGAACAAAGGGCAGCGAAACCGCGAGG

TTAAGCCAATCCCACAAATCTGTTCTCAGTTCGGATCGCAGTCTGCAACTCGACTGCGT

GAAGCTGGAATCGCTAGTAATCGCGGATCAGCATGCCGGGGGGAAAACCTTTCCCGGGG

CCTTGTACACACCGCCCGTCACACCACGAGAGTTTGTAACACCCGAAGTCGGTGAGGTA

ACCTTTTAGGAGCCAGCCGCCGAAGGTGGGACAGATGATTGGGGTG
```

FIGURE LEGENDS

**Figure 1. Phylogenetic relationship of strain QVS1 with B. subtilis strains.**
The phylogenetic tree of the *B. subtilis* QVS1 isolate (also referred to as SNW3) (A) and PCR amplified product of the surfactin encoding *srfA* gene (B). For panel (A), the 16S rRNA gene of the QVS1 isolate was sequenced and showed to be different compared to other *B. subtilis* strains reported. For panel (B), L-1: 1 kb ladder, C- : negative control, C+: positive control, 1: amplified product of *srfA* gene of *B. subtilis* QVS1. It is shown herein and in Example 1 that the QVS1 isolate is a newly identified micro-organism belonging to the *B. subtilis* species.

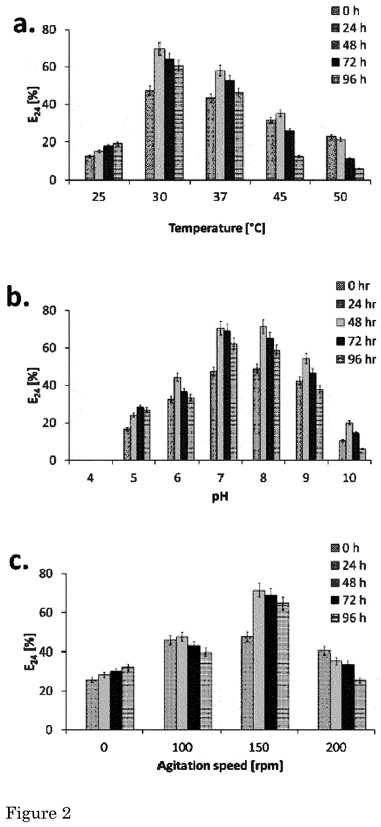

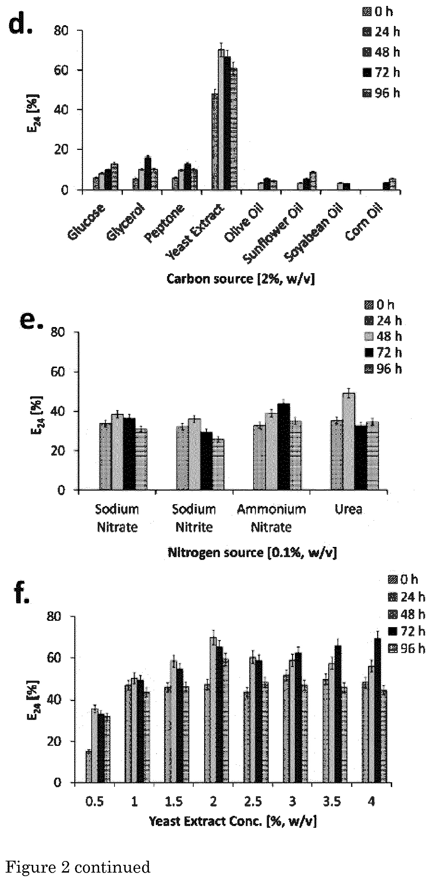

**Figure 2. Effect of culture conditions on emulsifying activity of the biosurfactants produced by *B. subtilis* QVS1.**
**a.** Temperature (°C); **b.** pH; **c.** Agitation speed (rpm); **d.** Carbon source (2%, w/v); **e.** Nitrogen source (0.1%, w/v); and **f.** yeast extract conc. (%, w/v). Figure 2 shows clear preferences for certain culture parameters measured by emulsifying activity. The error bars represent $\pm$ standard deviation of mean values obtained after three replicate experiments.

**Figure 3. Growth and biosurfactant production kinetics of bacterial isolate *B. subtilis* QVS1 under optimized culture conditions.**
Growth and biosurfactant production kinetics of the QVS1 isolate when cultured under beneficial fermentation conditions.

**Figure 4. Optimization of extraction method (upper) and extraction solvent system (lower) for recovery of biosurfactants from fermentation broth *of B. subtilis* QVS1.** In the upper graph, it is shown that acid precipitation followed by ethyl acetate extraction provides for the highest concentration of crude biosurfactant. The lower graph indicates that an extraction solvent system that further comprises methanol may provide for higher crude weight biosurfactant after extraction, and that the use of ethyl acetate as a sole extraction solvent provides for significantly higher ODA values which corresponds to more specific biosurfactant product recovery. The error bars represent $\pm$ standard deviation of mean values obtained after three replicate experiments. The same alphabetical letters above bars indicate no significant difference between or among the variables at 95% probability (*P<0.05*).

**Figure 5. Stability of crude biosurfactant at varying: (a) pH, (b) temperature, and (c) salt concentrations.**

Figure 5 shows in panels a.-c. that the crude biosurfactant produced by *B. subtilis* QVS1 is unexpectedly stable (in terms of surface tension and emulsifying activity) over a broad range of pH, temperature and salinity values.

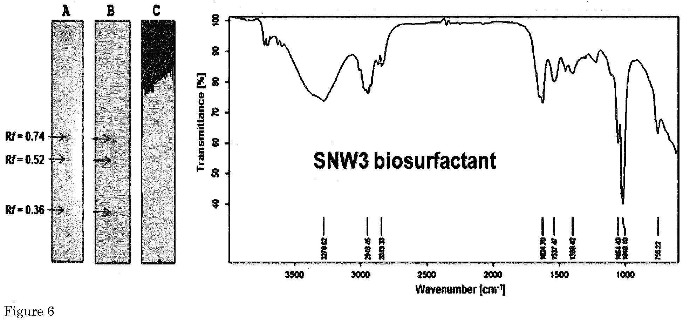

**Figure 6. TLC profile (left) and FTIR spectrum (right) of the partially purified biosurfactant produced by *B. subtilis* QVS1.**
Figure 6 illustrates TLC patterns and an FTIR spectrum of the purified biosurfactants produced by *B. subtilis* QVS1. Both the TLC pattern and FTIR spectrum are indicative of lipopeptide biosurfactant production, which is a characteristic property of *Bacillus* species.

**Figure 7. Biosurfactants of B. subtilis QVS1 have antibacterial activity.**
TLC (A, B, D, E) and antibiogram (C) of crude biosurfactant produced by *B. subtilis* QVS1 strain shows antibacterial activity against methicillin resistant *Staphylococcus aureus* (MRSA). Panels A and B: TLC plates showing UV active spots marked with lead pencil. Panel C: Antibiogram of TLC-separated QVS1 biosurfactant against *S. aureus* grown on nutrient agar at 37°C for 24 h. Shown are inhibitory zones around respective spots on TLC plate. Panel D: TLC plates visualized after exposure to iodine vapors showing lipid containing components in the crude extract. Panel E: TLC plate visualized after staining with 1% ninhydrin regent showing amino acid or peptide containing compounds in the crude extract. Arrows indicate active spots showing antibacterial activity.

**Figure 8. ODA/E$_{24}$ values with alternative, single carbon sources.**
Shown are ODA values (first panel) when single carbon sources as compared to reference YE are used for fermentation with strain QVS1. Also shown are E$_{24}$ values (second panel) when single carbon sources as compared to reference YE for fermentation with strain QVS1. RB (red beans) show potential in replacing YE in growth medium.

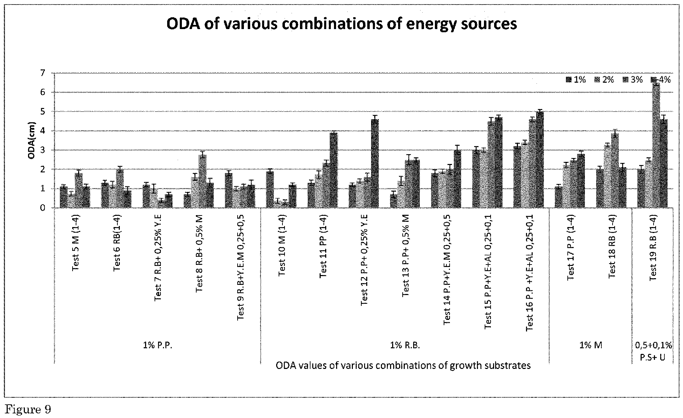

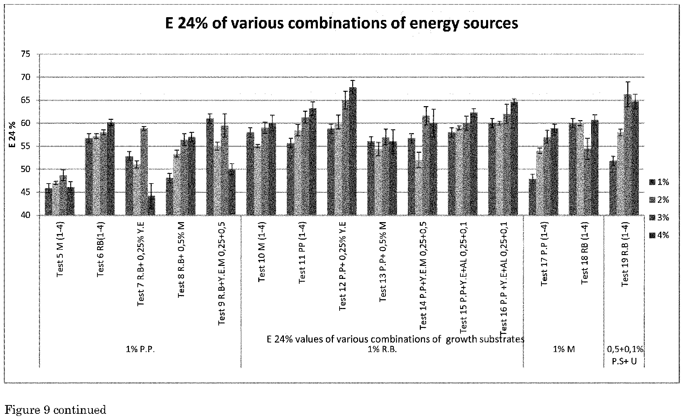

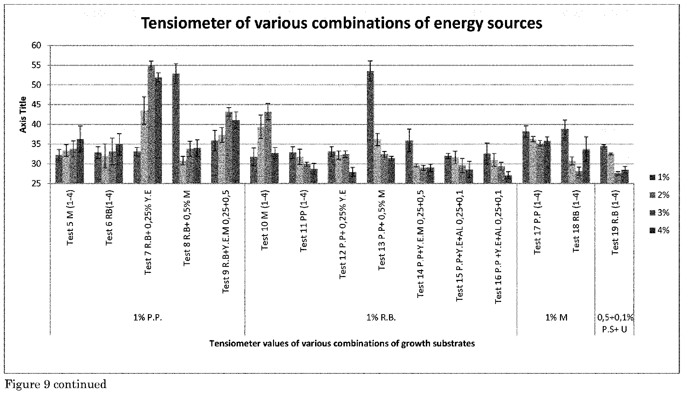

**Figure 9. ODA/E$_{24}$/SFT values with a combination of energy sources.** Figure 9 shows in three panels the effect of different combinations of energy sources in the QVS1 growth medium on ODA, E$_{24}$ and SFT (by tensiometer) values. RB, especially in test 19, represent a highly beneficial growth medium.

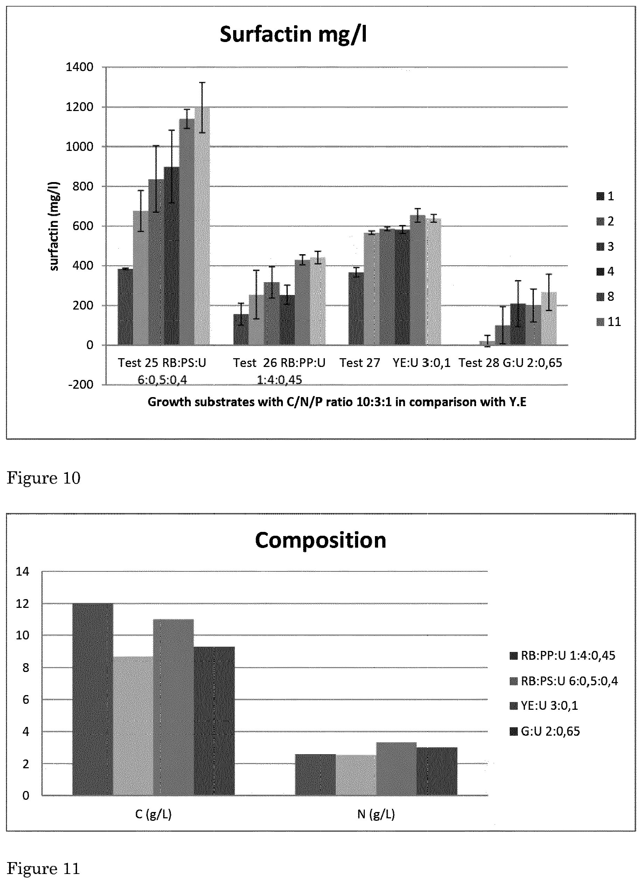

**Figures 10 and 11. Improved surfactin production after adjusting C:N:P ratio of growth substrates.**
Figure 10 exemplifies that an alternative growth substrate based on RB (red beans) and PS (potato starch) provides for highly beneficial surfactin production and outperforms the YE (yeast extract) control, when the alternative growth substrates are corrected for C:N:P ratio (10:3:1). Figure 11 confirms that approximately the same amount of carbon and nitrogen are provided by the alternative growth substrates.

**Figure 12. Surfactin and fengycin production with QVS1 strain**
Figure 12 shows that, with a C/N/P balanced cultivation media based on red beans, potato starch and urea (0.5% (w/v) PS + 6% (w/v) RB + 0.4% (w/v) U, with a C:N:P-ratio of 10:3:1), the QVS1 strain provides for surfactin and fengycin production with combined yields of more than 2 g/l shortly after day 5 of cultivation. Culture conditions were in short as follows: 23°C; pH 7; dissolved oxygen 6 mg/L; stirring was function of the oxygen level (200-500 rpm). Extraction was performed with ethanol, followed by centrifugation at 8000 rpm for 15 minutes at 20°C, further followed by storing at 4°C until measurement of concentration with LC-MS.

**Figure 13. Different types of red beans and surfactin production.** Different types of dried red beans have been tested in relation to surfactin production. It is shown that a culture medium based on beans effectively enhances surfactin production, and that RB1-RB5 effectively enhance surfactin production by QVS1 compared to white beans. RB1-RB5 relate to red beans produced by, or originating from, a plant of the species *Phaseolus vulgaris, Vigna angularis, Vigna umbellata* or *Abrus precatorius.* RB1 indicates speckled red beans originating from Pakistan, and RB2 indicates red beans (not speckled) originating from Belgium. Culture conditions were: shake flasks; 120 rpm; pH 6.5-7; 23°C.

EXAMPLES

**Example 1: Isolation and characterization of a novel Bacillus subtilis QVS1 strain for fermentative production of antimicrobial surface active lipopeptides (SALs).**

*Materials and methods*

Microorganism and culture conditions

The novel bacterial strain *Bacillus* sp. QVS1 was isolated from an oil field in Pakistan. After isolation, the bacterium was routinely cultured on nutrient agar and preserved on nutrient agar slants at 4 °C in a refrigerator for routine use. The bacterial strain was primarily characterized on the basis of Gram reaction, motility, spore and capsule formation. The physiological and biochemical characterization of the isolate was performed as per standard methods according to Bergey's Manual of Determinative Bacteriology (Holt JG, Bergey's Manual of Determinative Bacteriology: Lippincott Williams & Wilkins, Baltimore (1994)). The isolate was screened for biosurfactant producing ability using an agar plate-based oil spreading technique (JP05211892, Sakurai et al.).
The novel strain is termed *"Bacillus subtilis* QVS1" or "QVS1", and deposited with the Belgian Coordinated Collections of Microorganisms BCCM/LMG, Ghent, Belgium, under Accession Number "LMG P-30406" on 30 November 2017. This strain was also termed "SNW3".

Molecular characterization *of B. subtilis* QVS1

The bacterial genomic DNA was extracted from overnight pure culture of QVS1 strain using Norgen's bacterial genomic DNA isolation kit (Norgen Biotek Corp., Canada) according to the manufacturer's instructions. Molecular screening for *srfA* (Surfactin) gene was carried out through PCR using gene-specific primers (Table 1) in Thermocycler 2700 (Applied Biosystems, USA) according to Danashekar et al. (Int J Pharma Bio Sci 2: 497-504 (2011)). The PCR amplification cycle consisted of an initial denaturation step at 94 °C for 5 min, followed by 34 cycles of 25 sec at 94 °C and annealing at 54 °C for 40 sec. Afterwards, an elongation cycle (72 °C for 50 sec) was followed by a final extension step (72 °C for 6 min). The final PCR product (10 $\mu$L) was resolved with gel electrophoresis using 1 % agarose gel in 0.5× TBE buffer against 1 kb DNA marker (Fermentas) and ethidum bromide-stained bands were visualized under UV-transilluminator. The gel images were taken with Bio-Rad gel documentation system (Bio-Rad Laboratories, Inc., USA). All PCR reagents were provided by Promega Corporation (Madison, USA) and primers by e-Oligo (Hawthrone, NY, USA). The PCR reaction mixture consisted of 1 × green reaction buffer, 1.5 mM MgCl$_2$, 200 $\mu$M each dNTPs, 10 pM of each primer, 1.5 U Taq DNA polymerase and 5 $\mu$l of target DNA. The final reaction volume was 25 $\mu$l. The partial sequencing of the 16S rRNA gene was later carried out at the Genomic Division, Macrogen Inc., Seoul, Korea, using universal amplification and sequencing primers (Table 1). The sequence thus obtained was then analyzed and compared with nucleotide sequence database available at the National Center for Biotechnology Information (NCBI) website using Basic Local Alignment Search Tool (BLAST) program. The evolutionary history of QVS1 strain was inferred using the Neighbor-Joining (NJ) method (Saitou et al., Molecular biology and evolution, 4:406-425 (1987)). The evolutionary distances were computed using the Maximum Composite Likelihood (MCL) method (Tamura et al., Proceedings of the National Academy of Sciences of the United States of America, 101:11030-11035 (2004)). Phylogenetic analyses were conducted in Molecular Evolutionary Genetics Analysis (MEGA) software (Version 4.0) (Tamura et al., Molecular biology and evolution, 24:1596-1599 (2007)).

**Table 1.** List of primers and their targets used in this study.

| Target Gene | Primer | Sequence of the primer | Amplicon length (bp) |
|---|---|---|---|
| *16S rRNA* | 27F | 5'-AGAGTTTGATCMTGGCTCAG-3' | |
| | 1492R | 5'-TACGGYTACCTTGTTACGACTT-3' | |
| | 518F | 5'-CCAGCAGCCGCGGTAATACG-3' | |
| | 800R | 5'-TACCAGGGTATCTAATCC-3' | |
| *srf*A | *srf*A-F | 5'-AGGCAAGCAAGCCTCTGGCG-3' | 580 bp |
| | *srf*A-R | 5-CTTGTCCGCACAGGCACCGT-3' | |

Cultivation conditions for growth and biosurfactant production

Various culture conditions were optimized for biosurfactant production by *Bacillus subtilis* QVS1 at 250 ml shake flask level experiments. The culture conditions included pH (2.0 to 10.0), temperature (25, 30, 37, 45 and 50 °C), agitation speed (0, 120, 150 and 200 rpm), carbon source (glucose, glycerol, peptone, yeast extract, olive oil, sunflower oil, soybean oil and corn oil), nitrogen source (sodium nitrate, sodium nitrite, ammonium nitrate and urea) and yeast extract concentration (0.5-4 % (w/v)). The optimum culture condition of one experiment was kept constant during the second experiment and so on. All the fermentation experiments were carried out using mineral salts medium (MSM). After 16-18 h of growth in nutrient broth, the culture was separated by centrifugation at 5,000 rpm and washed twice with saline. After a second centrifugation, the biomass pellet was aseptically re-suspended into MSM and adjusted to an optical density (660 nm) of 1.0. This cell suspension was then used as inoculum in the biosurfactant production medium (MSM) at 5% (v/v) concentration. The MSM contained (g L$^{-1}$ of deionized water): $Na_2HPO_4$, 2.2; $KH_2PO_4$, 1.4; $MgSO_4.7H_2O$, 0.6; $FeSO_4.7H_2O$, 0.01; NaCl, 0.05; $CaCl_2$, 0.02; and 0.1 mL of trace elements solution containing (g L$^{-1}$ of deionized water): $ZnSO_4.7H_2O$, 2.32; $MnSO_4.4H_2O$, 1.78; $H_3BO_3$, 0.56; $CuSO_4.5H_2O$, 1.0; $NH_4MoO_4.2H_2O$, 0.39; KI, 0.66. The pH of the medium was adjusted to 7.0 $\pm$ 0.2 using 1M HCl and 1M NaOH and was autoclaved at 121 °C and 15 lb pressure for 20 min. The carbon and nitrogen sources were separately sterilized and added to the production medium at 2% and 0.1% concentration, respectively. All the chemicals were purchased from Sigma (Sigma-Aldrich®, USA).

Optimization of biosurfactant extraction from culture broth for analytics

The 72 h old culture broth was centrifuged at 12,000 rpm (15 min, 4 °C) and filtered (0.45 $\mu$m syringe filters). The cell free supernatant (CFS) was acidified with 6M HCl to pH 2 and left overnight at 4 °C. After centrifugation, the precipitates were collected and re-dissolved in phosphate buffer at pH 7. The biosurfactants were then extracted three times with equal volume of ethyl acetate as well as different ratios of an ethyl acetate/methanol combination (1:1, 2:1, 5:1 and 10:1) and pooled. The solvent was evaporated by using rotary evaporator at 45 °C. The remaining brown extract served as crude biosurfactant (also referred to as crude herein) and was used in different assays as *inter alia* described below.

Stability of the crude biosurfactant

The effect of a wide range of pH (1-11), temperature (25-121 °C), and salt concentrations (1-7%) on the activity and stability of the crude biosurfactant was tested. Briefly, the crude biosurfactant was dissolved in a range of pH buffers (pH 1, 3, 5, 7, 9, 11) and incubated for 1 h. Subsequently, the crude biosurfactant, dissolved in the appropriate buffer, was exposed to different temperature treatments (at 25, 40, 60, 80, 90, 100 °C) in a water bath for 1 h and also autoclaved at 121 °C under 15 lb pressure. Different concentrations of NaCl (1, 2, 3, 4, 5, 6 and 7%) were added to the crude biosurfactant solution prepared in distilled H2O and incubated for 1 h at room temperature. The activity and stability of the biosurfactant was determined after each treatment in terms of surface tension, emulsification index and oil displacement activity.

Analytical methods

The qualitative and quantitative analysis of the cell free supernatant (CFS) culture broth was achieved by biomass analysis, oil displacement assay, emulsification index ($E_{24}$) and surface tension measurement. For biomass analysis, the samples of the culture broth taken in a pre-weighed vial were centrifuged at 10,000 rpm for 15 min and clear supernatant solution was separated. The pellet (biomass) was then washed twice with distilled water and again centrifuged at 10,000 x g. After removing the water, the vial containing the cell pellet was placed in a drying oven (70 °C, 1 h). After drying, the bacterial dry weight was determined by subtracting the weight of the pre-weighed vial from that of vial's weight after drying.

The oil displacement activity (ODA) was determined by an assay as described by Rodrigues et al. (Colloids and Surfaces B: Biointerfaces 49:79-86 (2006)). Briefly, 40 μL of crude oil was added to a large clean petri plate (15 cm) filled with 50 mL of distilled water. A thin film of crude oil was developed. 20 μL of CFS was then added to the center of the oil film, and observed for a clear zone of oil displacement after 30 sec and measured. Appearance of this zone was considered as a positive test for biosurfactant production while the zone size measured in centimeters was indicative for the biosurfactant concentration.

The emulsification index ($E_{24}$) was determined as described by Cooper and Goldenberg (Applied and Environmental Microbiology 53:224-229 (1987)). Concisely, equal volume of CFS and kerosene were added to a test tube and mixed well with the vortex for 2 minutes. The mixture was allowed to stand for 24 h at room temperature and we then measurement percent emulsification as calculated with Eq. (1):

$$E_{24}(\%) = \frac{eHT}{tHT} \times 100 \qquad (1)$$

Where, 'eHT' is the emulsion height while 'tHT' corresponds to the total height of the solution.

The surface tension (SFT) of the cell-free supernatant was measured with a digital semi-automated tensiometer (Easy Dyne K20, KRUSS GmbH, Germany) using the standard Wilhelmy plate method at room temperature according to the manufacturer's instructions. All the measurements were taken as the mean of five measurements in mN m$^{-1}$ $\pm$ SD.

Physico-chemical characterization of the biosurfactant

Purification of biosurfactant by column chromatography.
Prior to physico-chemical characterization, the crude biosurfactant extract (1 g) was dissolved in methanol and was adsorbed on 1 g silica gel 60 (70-230 mesh Merck, Germany) and dried in a fume hood. The sample was then loaded on top of the glass column filled with silica gel 60 (230-400 mesh, Merck, Germany) along with a protective layer of 1.5 cm. The column loaded with crude biosurfactant was eluted with gradient change in mobile phase; starting with 100% n-hexane → 100% chloroform → chloroform/methanol (1:1 to 5:1) → ethyl acetate/methanol (1:1 to 5:1) → 100% ethyl acetate. Each fraction of 150 ml was collected and dried at 35°C in a rotary evaporator. In total 40 fractions were collected, each of 150 ml. After partial purification, the biosurfactants fractions of each microbial isolate were evaluated for chemical properties on a TLC plate under UV lamp after an 10% phosphomolybdic acid staining. The fractions showing similar spots on TLC plate were mixed and activity was determined using assays as described in analytical methods section.

Critical micelle dilution (CMD) and critical micelle concentration (CMC) of partially purified biosurfactants.
The cell-free supernatant of the samples produced by the QVS1 isolate were collected at different time intervals, and was diluted 10-folds up to three levels (i.e. 10x, 100x, and 1000x). The dilutions were labeled as CMD$^{-1}$, CMD$^{-2}$ and CMD$^{-3}$, respectively, and analyzed for surface tension values at room temperature by the Wilhelmy plate method using a tensiometer (EasyDyne K20, KRUSS, Germany). Likewise, the partially purified biosurfactants as described above were dissolved in de-ionized water at an initial concentration of 2 mg ml$^{-1}$ (w/v) and diluted accordingly. The surface tension of these biosurfactant solutions, i.e. cell-free fermentation broth and purified biosurfactant solution, was then measured in ascending order of concentration (i.e. lower to higher) at room temperature. All the measurements were done in triplicate; each value is represented as mean $\pm$ SD, and is expressed in mN m$^{-1}$.

Thin Layer Chromatography (TLC).
The preliminary chemical characterization of purified biosurfactant was done by thin layer chromatography (TLC). For this, 10 mg of the biosurfactant dissolved in 0.5 ml methanol was spotted on silica gel F254 TLC plates (Merck, Germany) using jet pointed capillary tube. The TLC plates were developed with chloroform/methanol/acetic acid (25:24:1) and allowed to air dry. The plates were then visualized after staining with 10% H$_2$SO$_4$ reagent. The plates were heated at 150°C on aluminum foil-coated hotplate until the spots appear (usually 5-10 min) and the R$f$ value of each spot was calculated.

Antibiogram of the TLC-purified SALs.
The antibiogram of the TLC-purified SALs was determined against Methicillin-resistant *Staphylococcus aureus* (MRSA). Briefly, the test bacterial culture was inoculated on to the surface of Muller-Hinton Agar plates and resolved TLC plates were placed in a way that front-side down, i.e. silica coated of the TLC plate touches the bacterial lawn. The plates were then incubated for 24 h at 37°C and were observed for zone of inhibition around the compounds resolved on TLC plate.

Fourier Transform Infra-Red-Attenuated Total Reflectance (FTIR-ATR) Spectrometry.

The IR spectrum of the crude and partially purified biosurfactant samples were recorded by using bench-top TENSOR 27™ Fourier Transform Infra-Red (FTIR) spectrometer (Bruker, Germany) equipped with universal PIKE-MIRacle™ Single Reflectance ATR accessory (PIKE Technology, Germany). The FTIR-ATR spectra were recorded at room temperature (approx. 25 °C) yielding an IR spectrum over the range of wave number 4000-600 cm$^{-1}$ with a speed of 10 scans per spectrum. All data were initially corrected for background spectrum and base line. The spectral measurements were taken in the transmittance (%) mode.

Statistical data analysis.

For statistical analyses, all the experiments were run in triplicates and mean values and standard deviation were calculated. The analysis of variance (ANOVA) followed by least significant difference (LSD) estimation at 95% confidence level, were computed by using Statistix software (version 8.1).

*Results*

Characterization of QVS1 isolate

The molecular characterization of the bacterial isolate QVS1 was carried out by standard morphological, biochemical and 16S rRNA sequence homology methods. The bacterial isolate QVS1 was found to be a gram positive, spore forming, motile and capsulated bacterium which showed positive tests for starch, lipid and casein hydrolysis, nitrate reduction, catalase, VP and citrate utilization while Methyl red test was negative and the fermentation of all sugars tested (lactose, dextrose, sucrose and manitol) was positive (Table 2).

The results of morphological and biochemical assays characterized the strain as member of Genus Bacillus, more specifically the *Bacillus subtilis*. The identification was confirmed on the basis of molecular characterization of the bacterial isolate by 16S rRNA sequence homology *(vide* Figure 1A). A molecular screening for *srfA* (surfactin synthase) gene was carried out and the gene was successfully amplified in the *B. subtilis* QVS1 isolate, having an amplicon size of 580 bp *(vide* Figure 1B).

**Table 2.** Biochemical characteristics of the bacterial isolate QVS1.

| Test | | Result |
|---|---|---|
| **Morphological features** | | |
| Gram reaction | | + |
| Shape | | Rod-shaped |
| Motility | | + |
| Sporulation | | + |
| Capsulation | | + |
| **Biochemical features** | | |
| Gelatin liquefaction | | + |
| Starch hydrolysis | | + |
| Lipid hydrolysis | | + |
| Casein hydrolysis | | + |
| H$_2$S production | | - |
| Triple sugar iron | Slant | + |
| | But | - |
| | Growth | + |
| Nitrate reduction | | + |
| Catalase test | | + |
| Urease test | | - |
| Oxidase test | | - |
| Indol test | | + |

(continued)

| Biochemical features | | |
| --- | --- | --- |
| Methyl red test | | - |
| Voges prausker test | | + |
| Citrate utilization | | + |
| Fermentation | L | + |
| | D | + |
| | S | + |
| | M | |

**Note:** +, positive test; -, negative test; L, lactose; D, dextrose; S, sucrose; M, maltose

Culture conditions for biosurfactant production by *B. subtilis* QVS1

Figures 2A-2F illustrate the effect of different culture conditions on biosurfactant production by *B. subtilis* QVS1 strain. Maximum biosurfactant production in terms of emulsifying capability was achieved at 30°C followed by 37 > 25 > 45 > 50 °C after 48 h of incubation as indicated by significantly different (P<0.05 and P<0.01) emulsifying activities of 69.67%, 58%, 15%, 35% and 21%, respectively (Figure 2A). In general, the bacterial isolate revealed significant growth and biosurfactant production in the range of mesophilic temperature, i.e. 30 to 37 °C.

The maximum biosurfactant production was achieved at a pH of 7 and 8 ($E_{24}$ = 70.5% and 71.4%, respectively). Deviating from this range significantly affected the biosurfactant production by the *B. subtilis* QVS1 strain. Drastic effects of low and high pH values (<5 and ≥9) were observed on the growth and biosurfactant production ability of the isolate *(vide* Figure 2B). It is known that the pH most suitable for biosurfactant production varies between different *B. subtilis* isolates.

Further, the maximum production was achieved when the fermentation experiments were carried out particularly at an agitation speed of 150 rpm ($E_{24}$ = 71%) during 48 h of incubation. On the other hand, the biosurfactant activity at 0, 100 and 200 rpm was observed during 48 h was significantly low (P<0.01) with emulsifying indices of 28, 47 and 35%, respectively *(vide* Figure 2C).

Interestingly, yeast extract was by far the best performing carbon source for the QVS1 isolate in terms of emulsifying capability *(vide* Figure 2D).

Production of the biosurfactants under optimized conditions

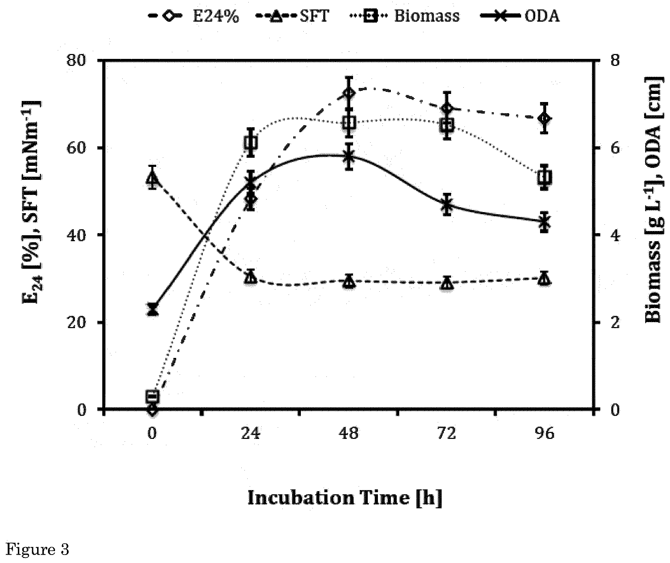

The study of growth and biosurfactant production kinetics of *B. subtilis* QVS1 at optimal conditions (30°C, 150 rpm, 5% inoculum size, pH 8, yeast extract (2%, w/v) and urea (0.1%, w/v), while recovery of the biosurfactant is achieved using ethyl acetate as extraction solvent) in 5L shake flask fermentation setup revealed a growth-associated production *(vide* Figure 3). A growth-associated biosurfactant production may facilitate substrate adsorption and metabolism by the bacterial cells. Under optimal culture conditions, the strain resulted in maximum biosurfactant production as evidenced by significantly higher emulsifying activity ($E_{24}$= 72.43%) and biomass (6.57 g $L^{-1}$) attaining maximum ODA (5.8 cm) as well as lowest surface tension (≈29.11 mN $m^{-1}$) during 48 h of the incubation *(vide* Figure 3). The lowest surface tension depicting critical micelle concentration (CMC) of the biosurfactant was achieved during initial 24 h of the incubation, which then remained constant throughout the fermentation cycle.

Optimization of biosurfactant recovery

For any biotechnological process, downstream processing is accountable up to 60% of the total production cost. Therefore, preferably, most of the biosurfactants must have to be recovered by using, either cost-effective recovery methods, or otherwise used as cell-free culture broths or crude preparations. The most frequently used and economical approaches for the retrieval of the biosurfactants include precipitation using acids, foam fractionation, or a combination thereof. Alternatively, solvent extraction by using many types of organic solvents is preferred due to amphiphilic nature and higher grade of purity of the biosurfactants. So far, the most successful, but toxic, solvent combination reported for recovery of different amphiphiles involves a combination of chloroform and methanol in different ratios.

In the current study, four different recovery methods viz; acid precipitation, extraction with n-hexane, ammonium sulfate precipitation and acid precipitation followed by organic solvent extraction using ethyl acetate were studied. Maximum recovery of the biosurfactant was attained when cell-free fermentation broth was treated with acid precipitation followed by ethyl acetate extraction (Figure 4). Thereafter, different ratios of ethyl acetate with methanol were evaluated in order to optimize the recovery and purity of the biosurfactants based on crude weight, surface tension, emulsifying index and oil displacement activity. Figure 4 shows that maximum crude weight ($\approx$1.798, g L-1) was obtained with ethyl acetate/methanol (2:1, v/v); however, the biosurfactant concentration was low as indicated by a low ODA (2.4 cm). Conversely, the ethyl acetate as a sole solvent could recover low crude weight ($\approx$1.134, g L-1) but showed significantly higher ODA value (10.21 cm) corresponding to specific product recovery of the biosurfactants.

Stability of the crude biosurfactant

Stability of the biosurfactants produced by *B. subtilis* QVS1 was evaluated over a broad range of temperature, pH and salinity as a function of emulsification, surface tension and oil displacing activity (Figure 5). The crude extract showed maximum emulsification activity and lowest surface tension values (i.e. 67 to 69% and 28 to 29 mN m$^{-1}$, respectively), when the pH, temperature and salt concentration were maintained as pH 7.0, 25°C and 2% (w/v), respectively. The stability results also indicated that the biosurfactants produced by the QVS1 strain were also stable at a pH range between 5 to 9 ($E_{24}$, 56-63%; SFT, 28-32 mN m$^{-1}$), temperature range between 25 to 100°C ($E_{24}$, 50-69%; SFT, 28-34 mN m-1), and between 1 to 6% (w/v) salt concentration ($E_{24}$, 62-68%, SFT; 28-31 mN m$^{-1}$). Precisely, the lowest surface tension values (28.4$\pm$0.1, 28.5$\pm$0.2, and 28.1$\pm$0.15) were observed at pH 7, 25°C and 2% (w/v), respectively, while maximum product destabilizations were recognized at acidic pH (pH 1 to 3), high temperature under pressure (121°C, 151b) and salt concentration of more than 6% (w/v) suggesting structural alterations or instability of one or other co-existing isomers of the biosurfactants. The stability of biosurfactant produced by B. *subtilis* QVS1 showed that it maintains its activity even at extreme physical and chemical stress conditions tested. The world's highest marine salinity is recorded to be approximately 3%, which also suggests possible use of the biosurfactants obtained in the present study for bioremediation of oil contaminated marine environments.

The reduction of surface tension is indicative of the biosurfactant production, thus its measurement would be the method of choice to quantify biosurfactant production. The minimum concentration required to achieve lowest surface tension, referred to as critical micelle concentration (CMC), further demonstrates formation of a stable emulsion (Silva et al., Colloids and Surfaces B: Biointerfaces, 79:174-183 (2010)). Therefore, the concentration of the biosurfactant in fermentation medium could be estimated by using the CMC and critical micelle dilution (CMD) techniques. Competent surfactants require less surfactant concentration to decrease surface tension i.e., have very low CMC values (Silva et al., Colloids and Surfaces B: Biointerfaces, 79:174-183 (2010); George et al., Applied Biochemistry and Biotechnology, 158:694-705 (2009); Pansiripat et al., Biochemical Engineering Journal, 49:185-191 (2010)).

As shown in Table 3, the CMD of the cell-free broth of *B. subtilis* QVS1 appeared comparatively stable up to 3-fold dilutions. This indicated that the isolate produced significant amount of biosurfactant. The critical micelle concentration (CMC) of the partially purified biosurfactants were found to be $\geq$ 610 mg/ml that was very high as compared to those previously reported for different lipopeptides (surfactin, iturin, and fengycin, having CMC values of 10, 20, and 11 mg L$^{-1}$, respectively) produced by *Bacillus* species (Korenblum et al., BMC microbiology, 12:252 (2012)).

**Table 3.** Relationship between surface tension and critical micelle dilution (CMD) of the cell free broth *of B. subtilis* QVS1 strain grown in MSM under optimized culture conditions.

| Time (h) | SFT$\pm$SD[†] (mN m$^{-1}$) | CMD$^{-1}\pm$SD[‡] (mN m$^{-1}$) | CMD$^{-2}\pm$SD (mN m$^{-1}$) | CMD$^{-3}\pm$SD (mN m$^{-1}$) |
|---|---|---|---|---|
| NC[§] | 71.34$\pm$0.02 | 71.33$\pm$0.02 | 71.36$\pm$0.02 | 71.36$\pm$0.02 |
| 0 | 44.20$\pm$0.15 | 46.25$\pm$0.51 | 53.43$\pm$0.01 | 67.82$\pm$0.05 |
| 24 | 30.21$\pm$0.63 | 31.65$\pm$0.12 | 34.45$\pm$0.22 | 49.64$\pm$0.03 |
| 48 | 29.52$\pm$0.05 | 30.51$\pm$0.01 | 31.42$\pm$0.02 | 32.23$\pm$0.02 |
| 72 | 28.04$\pm$0.32 | 29.68$\pm$0.03 | 30.05$\pm$0.01 | 32.01$\pm$0.03 |
| 96 | 29.33$\pm$0.02 | 30.08$\pm$0.21 | 31.04$\pm$0.01 | 32.84$\pm$0.03 |
| 120 | 29.25$\pm$0.01 | 30.75$\pm$0.08 | 31.82$\pm$0.31 | 33.63$\pm$0.05 |

[†] Average surface tension $\pm$ standard deviation (mN m$^{-1}$) of triplicate experiments.

[‡] Average critical micelle dilution (CMD)$\pm$ standard deviation (mN m$^{-1}$) of triplicate experiments. CMD$^{-1}$= 10x dilution; CMD$^{-2}$= 100x dilution; CMD$^{-3}$= 1000x dilution.

[§] Negative control = Un-inoculated mineral salts medium (MSM)

Characterization of the biosurfactants produced by the QVS1 strain.

The chemical characterization of the purified biosurfactants was done by using TLC and FTIR. Figure 6 illustrates TLC patterns and an FTIR spectrum of the purified biosurfactant. Three major spots on TLC plate with Rf value of 0.74, 0.52 and 0.36 became visible, when stained with iodine vapors and 1% ninhydrin reagent, while no spots on orcinol stained TLC plate were detected. This indicated the presence of nitrogen containing compounds. The chemical nature of purified biosurfactant was further evaluated by using FTIR coupled with universal attenuated total reflectance (ATR) accessory. The FTIR spectrum of the biosurfactant showing peaks in the frequency range of 3275.54 cm$^{-1}$ indicate NH2 stretch of the peptides, 800-600 cm$^{-1}$ peaks correspond to the C-C and C-H interactions of straight chain components, strong peaks in the range of aliphatic region (3000 to 2830 cm$^{-1}$) indicated asymmetric and symmetric stretches of CH3 and CH2, and 1055-1017 cm$^{-1}$ (C-O-C, C-O-P deformations), 1500-1450 cm$^{-1}$ (C-C=C asymmetric stretch of aromatic rings or H-C-H bend of alkanes), 1396.89 cm$^{-1}$ (combination CH and NH stretching bands of organic nitrogenous compounds) and 1221 cm$^{-1}$ (in-plane C-N bending or C-O ester linkages of aromatic rings). All the relevant peaks designated lipopeptide biosurfactants, the characteristic property of the *Bacillus* species. which produces *inter alia* lipopeptide class compounds such as surfactin, iturin and fengycin. The results of current study were in agreement with earlier reports on biosurfactants produced by different species of *Bacillus.*

Antibiogram of the TLC-purified SALs

The antibiogram of TLC-purified SALs of *B. subtilis* QVS1 strain against Methicillin-resistant *S. aureus* (MRSA) is shown in Figure 7. It was observed that TLC-purified SALs showed significant activity against MRSA.

**Example 2. Alternative growth substrates for enhanced production of biosurfactants by *Bacillus subtilis* QVS1.**

*Abstract*

The aim of the present study was to replace the costly yeast extract medium with more cost effective alternatives for biosurfactant production. Alternative growth substrates considered comprised potato peels, red beans, molasses and potato starch provided in mineral salt medium. These substrates were evaluated individually as well as in mixtures. Moreover, lipopeptide production was further enhanced by supplementation of additional inducers (urea, alanine). Biosurfactant production was assessed by determination of oil displacements, emulsification indexes and surface tension reductions. In general, mixtures of growth substrates performed better compared to single growth substrates with regard to biosurfactant production, although red beans in itself already seemed to provide for beneficial biosurfactant production. The best results were obtained with a mixtures of potato starch and red beans. Adjustment of the C/N/P-ratio of the medium to 10:3:1 further increased the yield of surfactin production till 1.2 g/l (versus 620 mg/L with yeast extract) The most promising alternative substrate consisted of 60 g/L red beans, 5 g/L potato starch and 4 g/L urea.

*Materials and methods*

Bacterial strain & culture media

The aforementioned *Bacillus subtilis* QVS1 strain was used in the experimentation described below.
From a glycerol (30%, -20°C) stock, the culture was grown on nutrient agar plates (Sigma Aldrich) and nutrient agar slants were used for short term storage at 4 °C. For inoculum preparation, fresh cells from a nutrient agar plate (Oxoid LTD, Basingstoke, Hampshire, England) were transferred to 100 ml nutrient broth (Oxoid LTD, CM0001) and incubated on a shaker at 30 °C for 24-48 hours. The cells were separated from the broth by centrifugation (12,000 rpm; 15 minutes) and the well pellets were re-suspended in fermentation medium (MSM).
Mineral salts medium (MSM) as described by Abouseoud et al. (Desalination, 223:143-151 (2008)) was used throughout the further experiments, and was supplemented by different additives. MSM contained (g/L of deionized water): 4.0 $K_2HPO_4$, 2.0 $KH_2PO_4$, 1.0 $MgSO_4.7H_2O$, 0.025 $FeSO_4.7H_2O$ 5.0 NaCl, 0.2 KCl, 0.02 $CaCl_2$, 0.5 urea, and 0.1 mL of trace elements solution containing (g/L of deionized water): 2.32 $ZnSO_4.7H_2O$, 1.78 $MnSO_4.4H_2O$, 0.56 $H_3BO_3$, 1.0 $CuSO_4.5H_2O$, 0.39 $NH_4MoO_4.2H_2O$, 0.66 KI. Adjustment of pH up to 7.0 $\pm$ 0.2 was realized using 1 M HCl and 1 M NaOH. This medium was supplemented with different growth substrates including yeast extract (YE, Oxoid (LP0021), molasses (M; purchased locally), red beans (RB, speckled red bean, purchased locally), potato peels (PP, steamed peel of different potato species purchased from potato processing industry) and potato starch (PS, Sigma Aldrich (S4251)), urea (U, Sigma Aldrich (U5378)), and/or amino acids alanine (AL, Sigma Aldrich). As a pretreatment step, the potato peel pulp (10.7 % dry matter) was homogenized with a spoon and frozen for a subsequently freeze drying step (Christ/Analis lyphilisator). Similarly, red beans (94.4% dry matter) were mixed in a blender (size reduction to mm size) and subsequently frozen and freeze dried before they were grinded to powder size. The material was stored at room temperature until use.

Experimental set-up biosurfactant production tests - screening test

Growth of strain QVS1 and the associated production of biosurfactants was examined via lab-scale shaking flasks (250 ml Erlenmeyers) experiments.

The flasks (triplicates) were filled with 44.6 mL of MSM and supplemented with specific growth substrates in different concentrations and combinations (see table 4) - test sets 1 to 19) reaching a final volume of 100ml. The QVS1 inoculum (2% = 2 mL per 100mL of a suspension of OD660= 1 and $1.29 \times 10^8$ cfu/ml) was added. After incubation times of 1, 2, 3 and 4 days at 30 °C (shaken at 150 rpm), bacterial cells were removed from the subsamples by centrifugation (12,000 rpm; 20 minutes) and the cell-free supernatant (CFS) was subjected to an oil displacement assay (ODA) and emulsification index % assay ($E_{24}$). Surface tension (SFT) measurement were performed only after 24h incubation time.

**Table 4.** Overview of growth substrates (g/100 mL) supplied in the different tests.

| | Test set | YE | PP | M | RB | PS | AL | Extra Urea | Glucose |
|---|---|---|---|---|---|---|---|---|---|
| Screening tests | | | | | | | | | |
| Single growth substrates | 1* | 1-5 | | | | | | | |
| | 2 | | 1-5 | | | | | | |
| | 3 | | | 1-5 | | | | | |
| | 4 | | | | 1-5 | | | | |
| Combinations with 1% PP | 5 | | 1 | 1-4 | | | | | |
| | 6 | | 1 | | 1-4 | | | | |
| | 7 | 0.25 | 1 | | 1-4 | | | | |
| | 8 | | 1 | 0.5 | 1-4 | | | | |
| | 9 | 0.25 | 1 | 0.5 | 1-4 | | | | |
| Combination with 1% RB | 10 | | | 1-4 | 1 | | | | |
| | 11 | | 1-4 | | 1 | | | | |
| | 12 | 0.25 | 1-4 | | 1 | | | | |
| | 13 | | 1-4 | 0.5 | | | | | |
| | 14 | 0.25 | 1-4 | 0.5 | 1 | | | | |
| | 15 | 0.25 | 1-4 | | 1 | | 0.1 | | |
| | 16 | 0.25 | 1-4 | | 1 | | 0.1 | 0.1 | |
| Combinations with 1% M | 17 | | 1-4 | 1 | | | | | |
| | 18 | | | 1 | 1-4 | | | | |
| Combinations with 0.5% PS+ urea 0.1% | 19 | | | | 1-4 | 0.5 | | 0.1 | |
| Biosurfactant production tests | | | | | | | | | |
| 'detailed tests' | 20 | | | | 3 | 0,5 | | 0.1 | |
| | 21 | | 4 | | 1 | | | | |
| | 22 | 0.25 | 4 | | 1 | | | | |
| | 23 | 3 | | | | | | | |
| | 24 | | | | | | | | 4 |
| Detailed tests with C:N:P = 10/3/1 | 25 | | | | 6 | 0.5 | | 0.45 | |
| | 26 | | 4 | | 1 | | | 0.4 | |
| | 27 | 3 | | | | | | 0.1 | |
| | 28 | | | | | | | 0.65 | 2 |

Experimental set-up biosurfactant production tests - screening test

Experimental set-up for biosurfactant production tests.

These lab scale tests were performed in triplicates as described for the screening experiments (30°C, shaken 120rpm), while subsamples were taken over time. The growth substrates considered are described in Table 4 (test sets 20-28). Samples for ODA, $E_{24}$ and SFT were taken as described above. For surfactin analysis, an aliquot of 2 ml broth was taken in a preweighed centrifugation tube, supplemented with 2 ml EtOH. All samples were mixed at room temperature (on rotary mixer) for 30-60 minutes, before separation of supernatant from the cells by centrifugation (8000 rpm, 15 minutes at 20°C). The supernatant was used for surfactin quantification.

Analyses

Oil displacement Activity (ODA) is an indicator for the presence of biosurfactants in cell free supernatant (CFS) and was performed as described earlier by Morikawa et al, 2000). Briefly, 40 ml of distilled water was added to an empty petri dish (15 cm diameter) followed by gentle placement of crude oil (10 $\mu$l) on the surface of water to form a continuous oil layer. Next, 10 $\mu$l of the CFS was added to the center of the uniform thin oil layer, and the diameter of clearing zones was measured in centimeters for quantitative analysis (Morikawa et al., Biochim Biophys Acta., 1488(3):211-8 (2000)).
Emulsification index ($E_{24}$) was used to quantify the emulsifying capacity of CFS and was essentially performed as described in Example 1. Briefly, 2 ml of kerosene was added in a test tube followed by 2 ml of CFS. Both were mixed at high speed by vortex, followed by a 24 h static period at room temperature. The $E_{24}$ index was calculated with the formula indicated in Example 1.
Surface tension (SFT) of CFS was determined using a KRUSS K20 digital Tensiometer (Kruss GmbH, Hamburg, Germany) and the Wilhelmy plate method (platinum plate), according to manufacturer's protocol. Prior to sample measurement surface tension of distilled water was adjusted as standard which is 71 mN/m. The SFT was measured in mN/m and performed at room temperature.
Direct Surfactant measurement was performed using LC/MS. Samples for surfactin quantification were analyzed by UPLC-Quattro PDA (MIE-OR-1021) equipped with column 18, 2.1 x 100 mm, 1.7 $\mu$m Waters Acquity BEH C18 with gradient program that consisted of solvent A (water + 0,1 % formic acid) and Solvent B (acetonitrile + 0,1 % formic acid) at a temperature of 41° C. Flow rate was 0.4 ml/min and 10 $\mu$ injection volume was provided. Crude biosurfactant was monitored at 195 nm.

*Results*

Evaluation of different single growth substrates for biosurfactant production.

In a first set of experiments, red beans (RB), molasses (M) and potato peels (PP) were tested as single alternative growth substrates for yeast extract (YE) that was tested along as reference condition. For each growth substrate parallel sets were initiated with concentrations ranging from 1% till 5% (w/v). The results of the ODA and $E_{24}$ measurements are summarized in Figure 8 and are an indirect indicator for the presence of biosurfactants. The effective production of biosurfactants (*inter alia* surfactin) was proven via TLC, FTIR and liquid chromatography and MS (data not shown).
Generally, a longer incubation time resulted in equal or higher ODA and $E_{24}$ measurements. Based on ODA measurements, 3% YE gave the best results (3-5 cm), especially after 4 days of incubation. In respect of the alternative substrates, 3-4% RB performed the best (about 2.5-2.8 cm), closely followed by 5% PP (2.2 cm). Molasses (M) was the least performing (< 2 cm). The emulsification index changed significantly with increasing substrate concentrations (increase of 10-20%). YE and RB performed equally well and better than PP. Again, M was performing the least which was also confirmed by limited surface tension reductions (SFT > 40 mN/m). The highest surface tension reduction was obtained with 3% YE (28.4 mN/m), followed by 2-3% RB.
Among the alternative growth substrates, RB, especially at 3%, performed best, followed by PP. With 3% RB, an ODA of 2.9 cm (versus 5.1 for 3% YE) and a significant surface tension reduction up to 30.6 mN/m (versus 28.4 mN/m for 3% YE) was recorded. Importantly, and unexpectedly, the emulsifying activity scored for RB as single carbon source is at least equally well as the emulsifying activity scored for YE as single carbon source.

Impact of combined growth substrates on surfactant production.

Next, the impact of combined growth substrates on surfactant production was tested. Different combinations were formed at different ratios (1:1, 2:1, 3:1, 4:1, 1:2, 1:3 and 1:4). Potato peel (1 to 4 % (w/v)) and red beans (1 to 4 % (w/v)) were combined with each other and also with molasses (1 to 4 %(w/v)). In addition, some combinations were supplemented at lower concentrations (0.1-0.5% (w/v)) with growth inducers including YE, M, PS, U, and AL. Each single main growth substrate at 1% (w/v) was combined with various other compounds as detailed in Table 4. Total growth substrate concentrations varied between 1 - 5.75 g per 100 ml.

Oil displacements data, data on emulsifying activity, and SFT data are summarized in Figure 9. ODA values of the reference condition (YE) were in the order of magnitude of 3-5 cm as shown in Figure 8A. For combined sources based on alternative growth substrates, ODA values ranged between 1- 6.5 cm, implying that some conditions exceeded the performance of the reference.

For the 1% PP based tests, the best co-substrates were 3% RB combined with 0.5% M (test condition 8) with ODA values up to 2.75 cm, an E24%-index ranging between 49- 56% and SFT measurements of 30-35 mN/m. When in addition 0.25% of YE was added (test condition 9), the results were negatively affected.

Best additives for the 1% RB based test conditions, were 4% PP + 0.25 % YE (test condition 12). Supplementation of this test condition with 0.5% AL (test condition 15) or 0.5% AL + 0.1% U (test condition 16) improved the ODA and surface tension reduction result even slightly (ODA from 4.6 to 5 cm; SFT from 28.4 to 27.1 mN/m), while the E24% index decreases slightly (from 66.6% to 64.6).

The 1% M based tests were performed only with 2 combinations as M was shown to be the least promising in previous tests. Nevertheless, in combination with PP and especially RB, M is not underperforming for inducing biosurfactant production. In these experiments, best additive was found to be 3% RB + 1% M (3+1), having an ODA of 3.8 cm, E24% index of 59% and a SFT value of 33 mN/m.

Further, one 0.5% (w/v) PS test condition was evaluated. In a pretest (data not shown), potato starch was used in different concentrations (0.1 - 1%) as alternative for potato peels in combination with 0.25% YE. In this pretest, 0.5% PS was found to perform the best. Therefore, 0.5% PS was selected as basis for test condition 19. Enhanced biosurfactant production was observed when 0.5% (w/v) PS was used in combination with 1-4% (w/v) RB. A very good biosurfactant production, even higher than the reference YE condition, was found with 0.5% PS + 3% RB + 0.1% U, which gave an ODA value of 6.5 cm, E24% index of 66.3 %, and SFE value of 27.6 mN/m. Again, semi-quantitative analyses (TL, FTIR) proved surfactin to be present as biosurfactant (data not shown).

Quantification of biosurfactant production for selected promising growth substrate

Based on the results of the screening tests, three good performing conditions (linked to test 19, test 11 and test 12) were selected for quantification of the biosurfactant concentration in function of time. The conditions with AL (test sets 15 and 16) were not considered as having added value because the effect of the extra supplement was not in proportion with the additional price of this additive. Two reference conditions were included in the tests, being 3% (w/v) YE and 4% (w/v) glucose. In the condition with glucose, a limited amount of surfactin was found (< 200 mg/L), while in the YE experiment 580mg/L was detected in the 4-day assay (Figure not shown). In the conditions with alternative growth substrates, lower surfactin concentrations were measured, ranging between 300 and 500 mg/L. The best performing alternative substrates were, similar like in the screening tests, the RB-PS-based test condition (test set 20). The fact that this test condition displayed higher ODA values in the screening tests compared to YE, but resulted in lower surfactin concentrations (500 mg/l versus 580 mg/L), may indicate that other biosurfactants than surfactin are formed. In the YE condition compared to the other test conditions, significantly high pH values were measured, that may have influenced the results.

For all the test conditions (test sets 20-24), the C:N:P-ratios were determined via elemental analyses of the different growth substrates and their proportional use in the test conditions. The results revealed that the nitrogen concentration (proportional to the present carbon amount) was lower in the test conditions with alternative substrates compared to 3% YE reference condition (data not shown).

Impact of C:N:P-ratios on biosurfactant production by *Bacillus subtillis* QVS1

New surfactin production tests were set-up (table 4, test sets 25-28) to verify the performance of the different substrates under similar C/N/P-ratios. The C/N/P-ratios were adjusted to 10:3:1 via addition of U, taking into account the soluble C-concentration. The results collected during the 11 day lasting tests are given in Figure 10. The 0.5% PS based test condition supplemented with 6% RB + 0.4% urea performed unexpectedly well with a gradual increase of surfactant from 400 mg/l at day 1, over 850 mg/L at day 4 and 1200 mg/L at day 11. The surfactin production in the presence of 3% YE was similar as in test 24 and stabilized after day 2 at 580-620 mg/L. Again, the glucose test performed less, which is explainable by the absence of proteins that may be needed to supply the essential amino acids required for surfactin production. The 1% RB based conditions with 4% PP + 0.45% U stabilized at 420 mg/L surfactin.

Conclusion

Surfactin production yields were obtained that exceeded those when using 3% YE as main substrate. The best performing medium was a combined substrate comprising of 0.5% (w/v) PS + 6% (w/v) RB + 0.4% U, resulting in a medium with a C:N:P-ratio of 10:3:1. It was highly unexpected that a medium based on RB and PS could provide for improved surfactin production as compared to a YE substrate.

**Claims**

1. A *Bacillus subtilis* deposited with the BCCM/LMG, Ghent, Belgium, under Acc. No. LMG P-30406, or a mutant *Bacillus subtilis* thereof.

2. A method for producing biosurfactants, comprising the step of:

   a) culturing a *Bacillus subtilis* according to claim 1 in a culture medium under culture conditions that allow for the production of biosurfactants;
   b) optionally, after step a), separating from said culture medium the *Bacillus subtilis* according to claim 1 so as to provide a cell-free culture medium comprising biosurfactants;
   c) optionally, recovering biosurfactants from said cell-free culture medium.

3. The method according to claim 2, wherein the culture medium is a liquid culture medium.

4. The method according to claim 2 or claim 3, wherein the culture medium comprises beans.

5. The method according to claim 4, wherein the culture medium further comprises a starch, and optionally a nitrogen source such as urea.

6. The method according to claim 5, wherein the starch is a potato starch.

7. The method according to any one of claim 2-6, wherein the culture medium comprises 0.1-40 wt.% beans and 0.01-25 wt.% starch.

8. The method according to any one of claims 2-7, wherein the culture medium comprises about 6 wt.% beans, about 0.5 wt.% potato starch and about 0.4 wt.% urea.

9. The method according to any one of claims 2-8, wherein the culture conditions that allow for the production of biosurfactants are a temperature of 27-40 °C, pH of 5-10 and/or an agitation speed of 100-200 rounds per minute (rpm).

10. The method according to any one of claims 2-9, wherein the step of recovering biosurfactants from said cell-free culture medium is performed by acid precipitation, followed by solvent extraction, preferably ethyl acetate extraction.

11. A composition comprising biosurfactants, obtainable by a method according to any one of claims 2-10.

12. The composition according to claim 11, comprising surfactin and fengycin.

13. The composition according to claim 11 or claim 12, having, or providing for, an

   (i) emulsification index ($E_{24}$) of at least 40%, preferably at least 50%, over a pH range of 5-9, and a surface tension of at most 40 mN m$^{-1}$, preferably at most 35 mN m$^{-1}$, over a pH range of 3-11;
   (ii) emulsification index ($E_{24}$) of at least 40%, preferably at least 45%, over a temperature range of 25-100 °C, and a surface tension of at most 35 mN m$^{-1}$ over a temperature range of 25-100 °C; and/or
   (iii) emulsification index ($E_{24}$) of at least 50% over a NaCl concentration range of 1-7% (w/v), and a surface tension of at most 33 mN m$^{-1}$ over a NaCl concentration range of 1-6% (w/v).

14. Use of a *Bacillus subtilis* according to claim 1 for producing biosurfactants.

15. Use of beans and starch in a culture medium of a biosurfactant-producing micro-organism for producing biosurfactants.

P113533EP00

Figure 1

Figure 2

Figure 2 continued

Figure 3

Figure 4

Figure 5

Figure 6

Figure 7

Figure 8

Figure 8 continued

Figure 9

**E 24% of various combinations of energy sources**

Figure 9 continued

**Tensiometer of various combinations of energy sources**

Tensiometer values of various combinations of growth substrates

Figure 9 continued

Figure 10

Figure 11

Figure 12

Figure 13

Europäisches
Patentamt

European
Patent Office

Office européen
des brevets

# EUROPEAN SEARCH REPORT

Application Number

EP 17 21 0008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/143316 A1 (HSIEH FENG-CHIA [TW] ET AL) 10 June 2010 (2010-06-10) | 11-13 | INV. C12P21/02 C12R1/125 C07K7/06 C07K7/64 C07K9/00 A01N63/00 |
| A | * the whole document * | 1-10,14, 15 | |
| X | WO 2014/178032 A1 (UNIV EAFIT [CO]; ASOCIACIÓN DE BANANEROS DE COLOMBIA AUGURA [CO]) 6 November 2014 (2014-11-06) | 11-13 | |
| A | * the whole document * | 1-10,14, 15 | |
| X | WO 02/26961 A2 (SHOWA DENKO KK [JP]; YONEDA TADASHI [JP]; MIYOTA YOSHIAKI [JP]; FURUYA) 4 April 2002 (2002-04-04) | 15 | |
| A | * the whole document * | 1-14 | |

TECHNICAL FIELDS
SEARCHED      (IPC)

C12P
C12R
C07K
A01N

The present search report has been drawn up for all claims

| Place of search | Date of completion of the search | Examiner |
|---|---|---|
| Berlin | 29 May 2018 | Schneider, Patrick |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 21 0008

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2018

| Patent document cited in search report | | | Publication date | Patent family member(s) | | | Publication date |
|---|---|---|---|---|---|---|---|
| US 2010143316 | A1 | | 10-06-2010 | NONE | | | |
| WO 2014178032 | A1 | | 06-11-2014 | BR | 112015027771 | A2 | 25-07-2017 |
| | | | | CN | 105357970 | A | 24-02-2016 |
| | | | | CR | 20150600 | A | 17-06-2016 |
| | | | | EP | 2991492 | A1 | 09-03-2016 |
| | | | | PH | 12015502522 | A1 | 29-02-2016 |
| | | | | US | 2016073642 | A1 | 17-03-2016 |
| | | | | WO | 2014178032 | A1 | 06-11-2014 |
| WO 0226961 | A2 | | 04-04-2002 | AT | 441707 | T | 15-09-2009 |
| | | | | AU | 9230401 | A | 08-04-2002 |
| | | | | EP | 1320595 | A2 | 25-06-2003 |
| | | | | KR | 20030059162 | A | 07-07-2003 |
| | | | | US | 2004043451 | A1 | 04-03-2004 |
| | | | | WO | 0226961 | A2 | 04-04-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 05211892 B, Sakurai

### Non-patent literature cited in the description

- **LANG S.** *Curr Opin Colloid Interface Sci,* 2002, vol. 7, 12-20
- **ZAJIC JE et al.** *CRC Crit Rev Biotechnol,* 1984, vol. 1, 87-107
- **MAIER RM.** *Adv Appl Microbiol,* 2003, vol. 52, 101-21
- **STELLER S et al.** *J Chromatogr B,* 2000, vol. 737, 267-75
- **LIN et al.** *Biotechnol. Prog.,* 1993, vol. 9, 138-145
- **GRANGEMARD et al.** *Appl. Biochem. Biotechnol.,* 2001, vol. 90, 199-210
- **DESAI et al.** *Microbiology and Molecular Biology Reviews,* 1997, vol. 61, 47-64
- **DANASHEKAR et al.** *Int J Pharma Bio Sci,* 2011, vol. 2, 497-504
- **SAITOU et al.** *Molecular biology and evolution,* 1987, vol. 4, 406-425
- **TAMURA et al.** *Proceedings of the National Academy of Sciences of the United States of America,* 2004, vol. 101, 11030-11035
- **TAMURA et al.** *Molecular biology and evolution,* 2007, vol. 24, 1596-1599
- **RODRIGUES et al.** *Colloids and Surfaces B: Biointerfaces,* 2006, vol. 49, 79-86
- **COOPER ; GOLDENBERG.** *Applied and Environmental Microbiology,* 1987, vol. 53, 224-229
- **SILVA et al.** *Colloids and Surfaces B: Biointerfaces,* 2010, vol. 79, 174-183
- **GEORGE et al.** *Applied Biochemistry and Biotechnology,* 2009, vol. 158, 694-705
- **PANSIRIPAT et al.** *Biochemical Engineering Journal,* 2010, vol. 49, 185-191
- **KORENBLUM et al.** *BMC microbiology,* 2012, vol. 12, 252
- **ABOUSEOUD et al.** *Desalination,* 2008, vol. 223, 143-151
- **MORIKAWA et al.** *Biochim Biophys Acta.,* 2000, vol. 1488 (3), 211-8